# EUROPEAN PATENT APPLICATION

(11) **EP 4 843 563 A2**
(43) Date of publication of application: **25.11.2026**
(21) Application number: 26207297.8
(22) Date of filing: 15.11.2016
(51) Int. Cl.: A61K 9/00

(54) **TREATMENT OF AGE-RELATED MACULAR DEGENERATION USING RNA COMPLEXES THAT TARGET MYD88 OR TLR3**

(30) Priority: 16.11.2015 US 201562255878 P
(62) Document number(s) of the earlier application(s) in accordance with Art. 76 EPC: 16865834.2 / 3 377 630
(71) Applicant: OliX Pharmaceuticals, Inc., Gyeonggi-do 16226 (KR)
(72) Inventor: LEE, Dong-ki, 137-776 Seoul (KR); HONG, Sun Woo, 45 Suwon-si (KR); HONG, Isu, 27 Seoul (KR); HWANG, Ji Hye, 420-721 Seoul (KR)
(74) Representative: Boxall, Sarah Jane

(57) **Abstract**

In certain aspects, provided herein are RNA complexes that inhibit Myeloid differentiation primary response gene 88 (MyD88) and/or Toll-like receptor 3 (TLR3) and are useful in the treatment of age-related macular degeneration (AMD). In certain aspects, provided herein are pharmaceutical compositions comprising such RNA complexes and methods of using such RNA complexes and pharmaceutical compositions.

## Description

### RELATED APPLICATIONS

This application claims the benefit of priority to U.S. Provisional Patent Application serial number 62/255,878, filed November 16, 2015, which is hereby incorporated by reference in its entirety.

### BACKGROUND

Age-related macular degeneration (AMD) is a disease that results from the degeneration of the retinal pigmented epithelium lining in the eye's macula, which leads to vision loss. The macula is a small area in the retina made up of the light-sensitive tissues lining the back of the eye and plays a critical role in central vision. AMD is one of the leading causes of blindness worldwide.

AMD occurs in "wet" and "dry" forms. Wet AMD is the result of abnormal blood vessel growth in the retina. In wet AMD, increased amount of vascular endothelial growth factor (VEGF) contributes to this neovascularization, so therapeutic options include the use of VEGF inhibitors are used. However, many patients treated with VEGF inhibitors develop geographic atrophy (GA), which is a primary symptom of late dry macular degeneration, within a few years of treatment. The disease pathogenesis of dry AMD is unclear and no medical treatment is currently available for dry AMD. Therefore, the development of therapeutics that can treat both wet and dry macular degeneration needed.

### SUMMARY

MyD88 and TLR3 play important roles in the onset of both dry AMD and wet AMD. Unlike VEGF antibodies, which are ineffective at treating dry macular degeneration, a therapeutic agent targeting MyD88 or TLR3 can be used to treat both wet and dry macular degeneration.

In certain aspects, provided herein are RNA complexes that inhibit Myeloid differentiation primary response gene 88 (MyD88) and/or Toll-like receptor 3 (TLR3) and are useful in the treatment of age-related macular degeneration (AMD) (*e.g.,* wet and/or dry AMD). In certain aspects, provided herein are pharmaceutical compositions comprising such RNA complexes and methods of using such RNA complexes and pharmaceutical compositions.

In certain aspects, provided herein is an RNA complex comprising an antisense strand having sequence complementarity to a MyD88 mRNA sequence (*e.g.,* a human MyD88 mRNA sequence) and a sense strand having sequence complementarity to the antisense strand. In some embodiments, the RNA complex is capable of inhibiting MyD88 expression by a cell. In certain embodiments, the RNA complex is capable of inhibiting MyD88 production by a cell. In some embodiments, the RNA complex is an asymmetric short interfering RNA (an asiRNA). In some embodiments, the RNA complex is a long asymmetric short interfering RNA (a lasiRNA).

In some embodiments, the antisense strand is at least 19 nucleotides (nt) in length. In some such embodiments, the antisense strand is 19 to 21 nt in length (*i.e.,* 19, 20 or 21 nt in length). In other such embodiments, the antisense strand is at least 24 nt in length (*e.g.,* 24 to 121 nt in length), e.g., 31 nt in length. In certain embodiments, at least 13, 14, 15, 16, 17, 18, 19, 20 or 21 nt of the antisense strand are complementary to the MyD88 mRNA sequence. In some embodiments, the sense strand is 15 to 17 nt in length (*i.e.,* 15 nt in length, 16 nt in length or 17 nt in length). In certain embodiments, at least 15 nt, at least 16 nt or at least 17 nt of the sense strand are complementary to the sequence of the antisense strand. Representative RNA complexes include the RNA complexes listed in Table 1, Table 2, Table 3, Table 4, Table 5 or Table 6.

In certain aspects, provided herein is an RNA complex comprising an antisense strand having sequence complementarity to a TLR3 mRNA sequence (*e.g.,* a human TLR3 mRNA sequence) and a sense strand having sequence complementarity to the antisense strand. In some embodiments, the RNA complex is capable of inhibiting TLR3 expression by a cell. In certain embodiments, the RNA complex is capable of inhibiting TLR3 production by a cell. In some embodiments, the RNA complex is an asymmetric short interfering RNA (an asiRNA). In some embodiments, the RNA complex is a long asymmetric short interfering RNA (a lasiRNA).

In some embodiments, the antisense strand is at least 19 nucleotides (nt) in length. In some such embodiments, the antisense strand is 19 to 21 nt in length (*i.e.,* 19, 20 or 21 nt in length). In other such embodiments, the antisense strand is at least 24 nt in length (*e.g.,* 24 to 121 nt in length). In certain embodiments, at least 13, 14, 15, 16, 17, 18, 19, 20 or 21 nt of the antisense strand are complementary to the TLR3 mRNA sequence. In some embodiments, the sense strand is 15 to 17 nt in length (*i.e.,* 15 nt in length, 16 nt in length or 17 nt in length). In certain embodiments, at least 15 nt, at least 16 nt or at least 17 nt of the sense strand are complementary to the sequence of the antisense strand. Representative RNA complexes include the RNA complexes listed in Table 7, Table 8 or Table 10.

In some embodiments, the RNA complex provided herein comprises a chemical modification, wherein the modification facilitates the penetration of a cellular membrane in the absence of a delivery vehicle. In some embodiments, the modification is a 2'-O-methylated nucleoside, a phosphorothioate bond and/or a cholesterol moiety. In some such embodiments, the 2'-O-methyl nucleoside is positioned at the 3' terminus of the sense strand. In some such embodiments, the 3' terminal region of the sense strand comprises a plurality of 2'-O-methylated nucleosides (*e.g.,* 2, 3, 4, 5 or 6 2'-O-methylated nucleosides within 6 nucleosides of the 3' terminus). In other embodiments, the 2'-O-methyl nucleoside is positioned at the 3' terminus of the antisense strand. In some such embodiments, 3' terminal region of the antisense strand comprises a plurality of 2'-O-methylated nucleosides (*e.g.,* 2, 3, 4, 5 or 6 2'-O-methylated nucleosides within 6 nucleosides of the 3' terminus). In some embodiments, both the 3' terminal region of the sense strand and the 3' terminal region of the antisense strand comprise a plurality of 2'-O-methylated nucleosides. Representative RNA complexes include the modified RNA complexes listed in Table 3, Table 4, Table 5, Table 6, Table 8 or Table 10. In certain embodiments, the RNA complex is not cytotoxic.

In some embodiments, the RNA complex provided herein comprises a phosphorothioate bond. In some embodiments, at least 25%, 30%, 35%, 40%, 45%, 50%, 55%, 60%, 65%, 70%, 75%, 80%, 85%, 90% or 95% of the bonds between the ribonucleotides in the sense strand of the RNA complex are phosphorothioate bonds. In some such embodiments, all of the bonds between the ribonucleotides in the sense strand of the RNA complex are phosphorothioate bonds. Similarly, in some embodiments, at least 25%, 30%, 35%, 40%, 45%, 50%, 55%, 60%, 65%, 70%, 75%, 80%, 85%, 90% or 95% of the bonds between the ribonucleotides in the antisense strand of the RNA complex are phosphorothioate bonds. In some such embodiments, all of the bonds between the ribonucleotides in the antisense strand of the RNA complex are phosphorothioate bonds.

In some embodiments, the RNA complex provided herein comprises a cholesterol moiety. In some embodiments, the cholesterol moiety is attached to the 3' terminus of the sense strand.

In certain aspects, provided herein is a pharmaceutical composition comprising an RNA complex provided herein and a pharmaceutically acceptable carrier. In certain embodiments, the pharmaceutical composition is formulated for administration to the eye (*e.g.,* as an eye drop). In some embodiments, the pharmaceutical composition is formulated for intravitreal delivery.

In certain aspects, provided herein is a method of inhibiting MyD88 and/or TLR3 expression by a cell comprising contacting the cell with an RNA complex and/or a pharmaceutical composition provided herein. In some embodiments, the cell is present in the eye of a human subject (*e.g.,* a human subject with wet or dry AMD). In certain aspects, provided herein is a method of treating a human subject for AMD (*e.g.,* wet AMD and/or dry AMD) comprising administering to the subject, e.g., to the eye, an RNA complex and/or pharmaceutical composition provided herein. In some embodiments, the RNA complex and/or a pharmaceutical composition is administered to the eye by intravitreal injection.

### BRIEF DESCRIPTION OF THE DRAWINGS

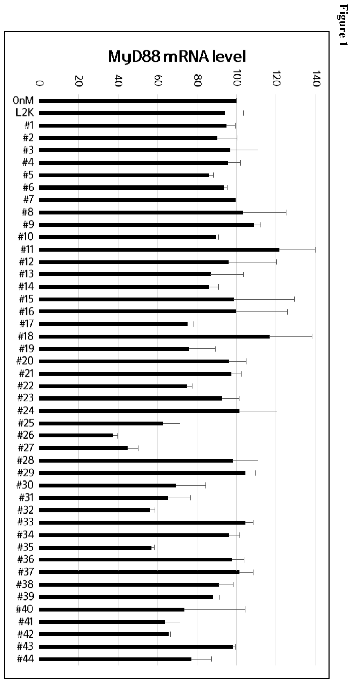

**Figure 1** shows the gene silencing efficiency of exemplary asiRNAs that target MyD88. The asiRNAs were transfected into HeLa cells at a concentration of 0.3 nM, and, after 24 hours, the degree of MyD88 mRNA expression was determined using real-time PCR. The mean and standard deviation of three repeat experiments are depicted.

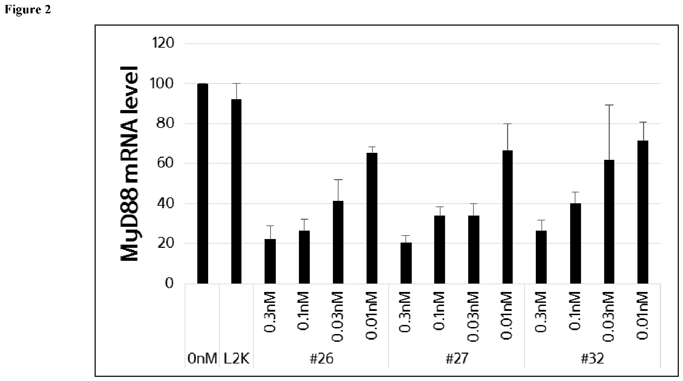

**Figure 2** shows the gene silencing efficiency of exemplary asiRNAs that target MyD88. The asiRNAs were transfected into HeLa cells at a concentration of 0.3 nM, 0.1 nM, 0.03 nM and 0.01 nM, and, after 24 hours, the degree of MyD88 mRNA expression was determined using real-time PCR. The mean and standard deviation of three repeat experiments are depicted.

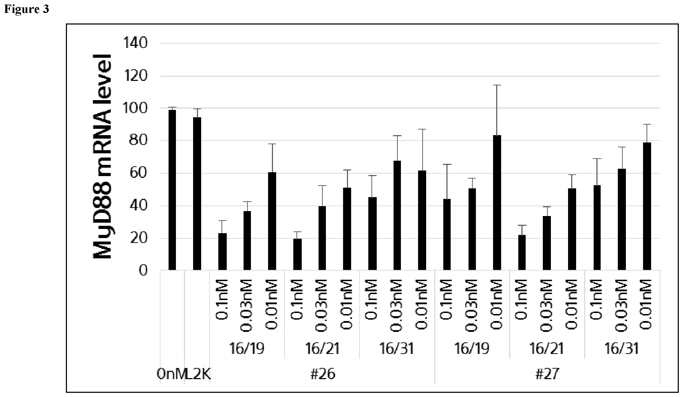

**Figure 3** shows the gene silencing effect of exemplary asiRNAs having different antisense strand lengths (19, 21 or 31 nucleotides) that target MyD88. The asiRNAs were transfected into HeLa cells at a concentration of 0.1 nM, 0.03 nM or 0.01 nM, and, after 24 hours, the degree of MyD88 mRNA expression was determined using real-time PCR. The mean and standard deviation of three repeat experiments are depicted.

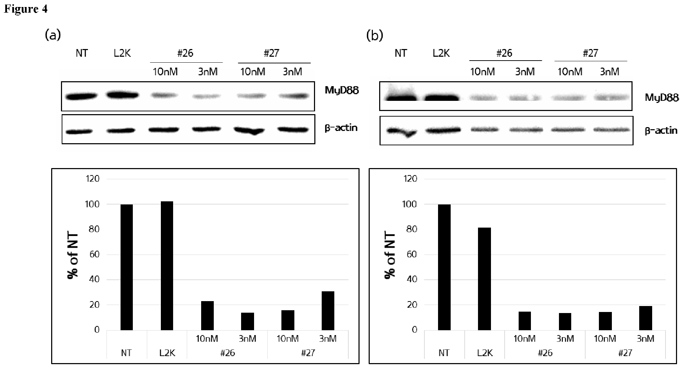

**Figure 4** shows the inhibition of MyD88 protein expression by exemplary asiRNAs that target MyD88. The asiRNAs were transfected into A549 or HeLa cells at a concentration of 3 nM or 10 nM, and, after 48 hours, protein was extracted and a western blot performed. Panel (a) depicts the MyD88 protein expression level in A549 cells 48 hours after transfection. Panel (b) depicts the MyD88 protein expression level in in HeLa cells 48 hours after transfection. (NT = no treatment, L2K = transfection control).

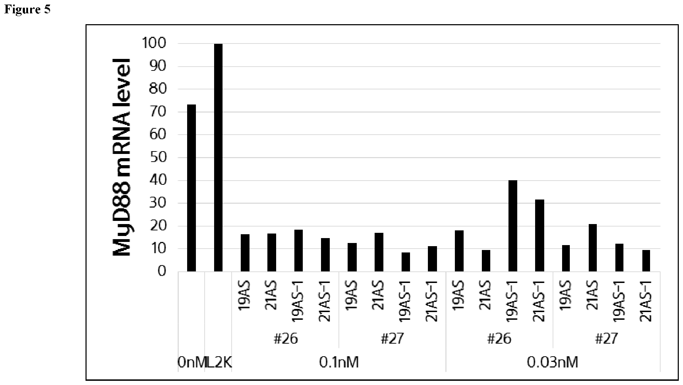

**Figure 5** shows the gene silencing efficiency of exemplary asiRNAs containing 2'-O-Methylation modifications. The asiRNAs were transfected into HeLa cells at a concentration of 0.1 nM or 0.03 nM, and, after 24 hours, the degree of MyD88 mRNA expression was determined using real-time PCR.

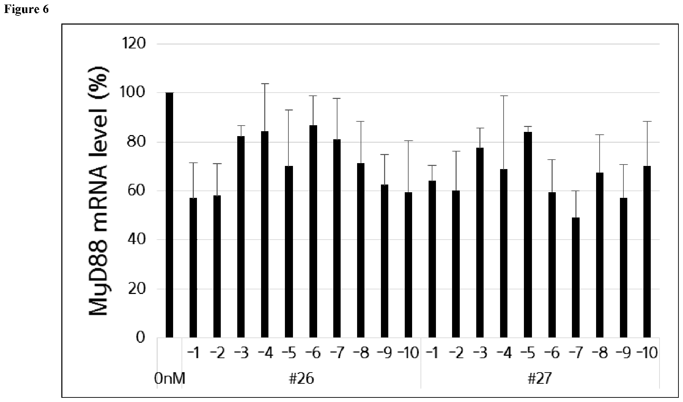

**Figure 6** shows the gene silencing efficiency of exemplary MyD88-targeting cell penetrating asiRNAs (cp-asiRNAs, or cp-asiMyD88s) to which various chemical modifications have been applied. The cp-asiRNAs were incubated without transfection vehicle in the presence of HeLa cells at a concentration of 1 µM and, after 48 hours, the degree of MyD88 mRNA expression was determined using real-time PCR. The mean and standard deviation of three repeat experiments are depicted.

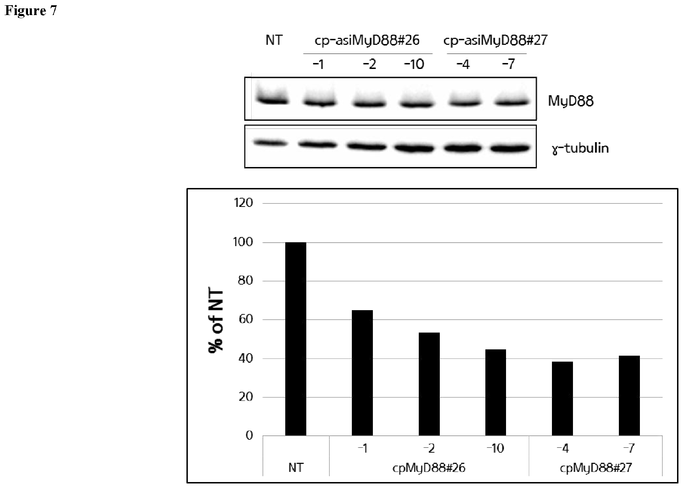

**Figure 7** shows the inhibition of MyD88 protein expression by exemplary cp-asiRNAs. The cp-asiRNAs were contacted to HeLa cells without transfection vehicle and, after 48 hours, protein was extracted and a western blot performed. (NT = no treatment).

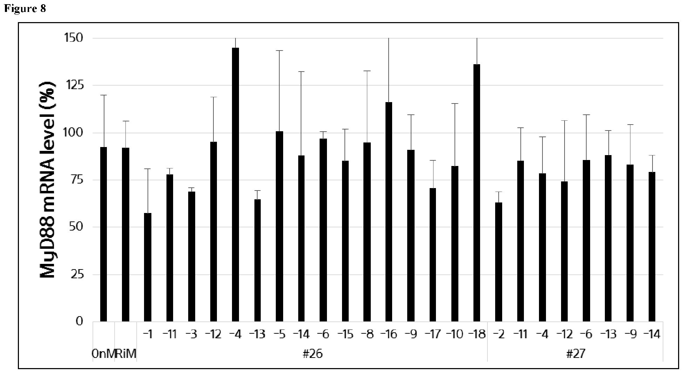

**Figure 8** shows the gene silencing efficiency of exemplary cp-asiRNAs to which various chemical modifications have been applied. The cp-asiRNAs were incubated without transfection vehicle in the presence of HeLa cells at a concentration of 1 µM and, after 48 hours, the degree of MyD88 mRNA expression was determined using real-time PCR. The mean and standard deviation of three repeat experiments are depicted.

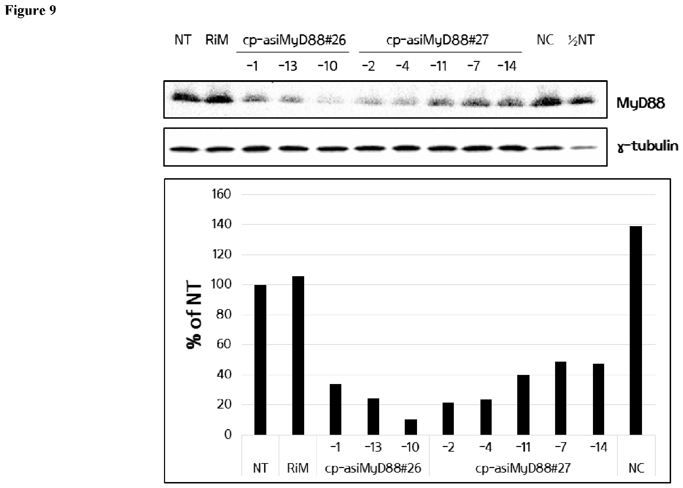

**Figure 9** shows the inhibition of MyD88 protein expression by exemplary cp-asiRNAs. The cp-asiRNAs were contacted to HeLa cells without transfection vehicle and, after 48 hours, protein was extracted and a western blot performed. (NT = no treatment, RiM = transfection reagent only, NC = negative control).

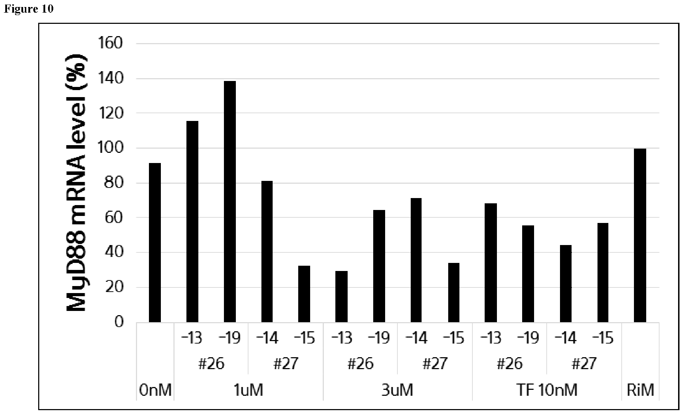

**Figure 10** shows the gene silencing efficiency of cp-asiRNAs having different antisense strand lengths (21 or 19 nucleotides) and containing 2'-O-Methylation modifications. Each cp-asiRNAs was incubated without transfection vehicle in the presence of HeLa cells at a concentration of 1 µM and, after 48 hours, the degree of MyD88 mRNA expression was determined using real-time PCR.

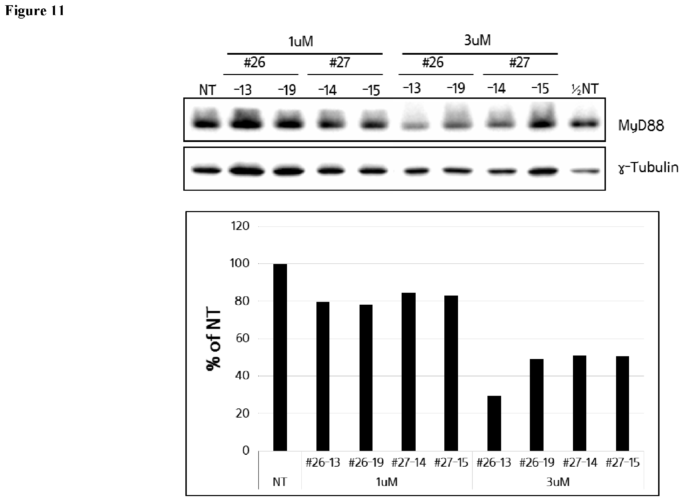

**Figure 11** shows the inhibition of MyD88 protein expression by exemplary cp-asiRNAs. The cp-asiRNAs were incubated without transfection vehicle in the presence of HeLa cells at a concentration of 1uM or 3uM and, after 48 hours, protein was extracted and a western blot performed. (NT = no treatment).

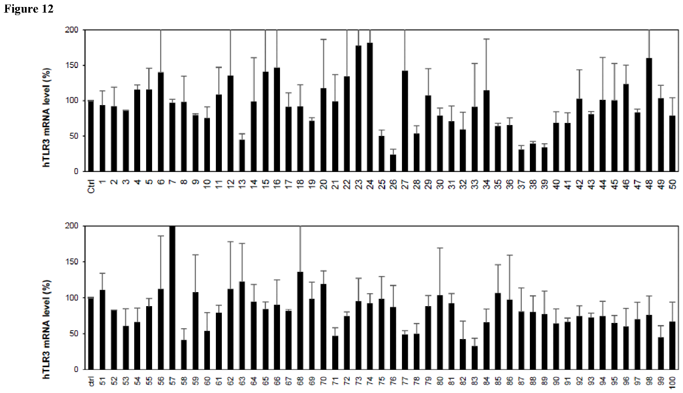

**Figure 12** shows the gene silencing efficiency of exemplary asiRNAs that target Toll-like receptor 3 (TLR3). The asiRNAs were transfected into HaCaT cells at a concentration of 0.1 nM, and, after 24 hours, the degree of TLR3 mRNA expression was determined using qRT-PCR. The mean and standard deviation of two repeat experiments are depicted.

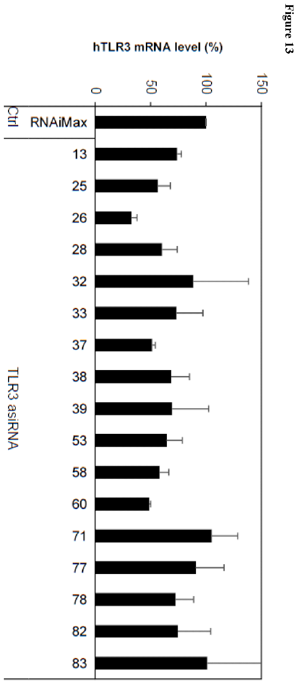

**Figure 13** shows the gene silencing efficiency of exemplary asiRNAs that target TLR3. The asiRNAs were transfected into HaCaT cells at a concentration of 0.1 nM and, after 24 hours, the degree of TLR3 mRNA expression was determined using qRT-PCR. The mean and standard deviation of two repeat experiments are depicted.

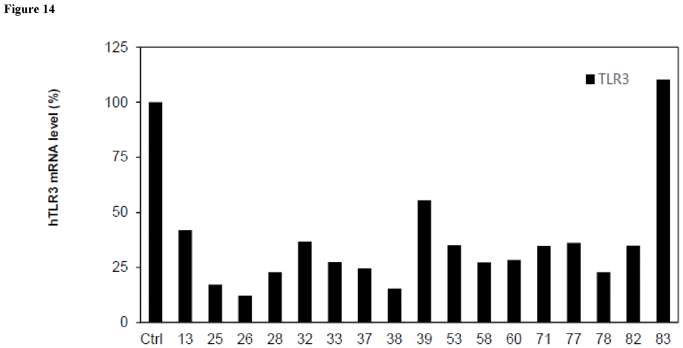

**Figure 14** shows the gene silencing efficiency of exemplary asiRNAs that target TLR3. The asiRNAs were transfected into HaCaT cells at a concentration of 0.3 nM and, after 24 hours, the degree of TLR3 mRNA expression was measured using qRT-PCR.

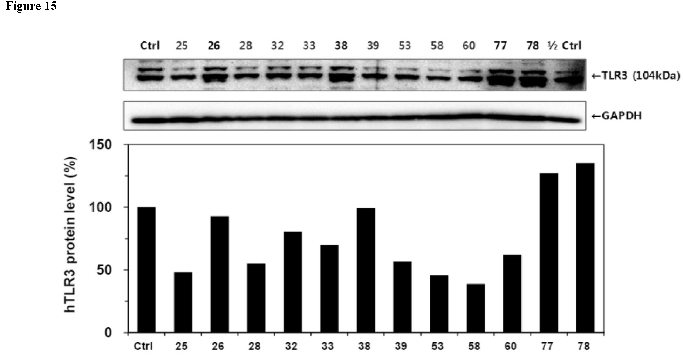

**Figure 15** shows the inhibition of TLR3 protein expression by exemplary asiRNAs. The asiRNAs were transfected into HaCaT cells at a concentration of 10 nM and, after 48 hours, the degree of TLR3 protein expression was determined using western blot.

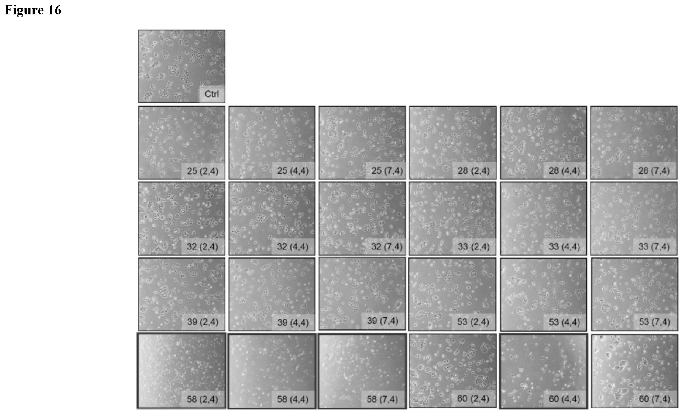

**Figure 16** shows images of HaCaT cells treated by exemplary cp-asiRNAs for 24 hours. The cp-asiRNAs were incubation of 1uM and, after 24 hours, the morphology of the HaCaT cells was imaged by ECLIPSE 100 (Nikon).

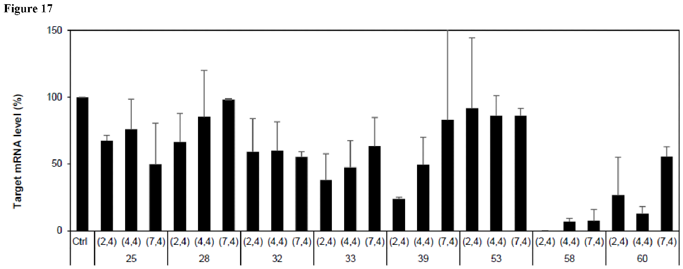

**Figure 17** shows the gene silencing efficiency of exemplary TLR3-targeting cell penetrating asiRNAs (cp-asiRNA, or cp-asiTLR3s) to which various chemical modifications have been applied. The cp-asiRNAs at a concentration of 1µM were incubated with HaCaT cells and, after 48 hours, the degree of TLR3 mRNA expression was measured using qRT-PCR. The mean and standard deviation of two repeat experiments are depicted.

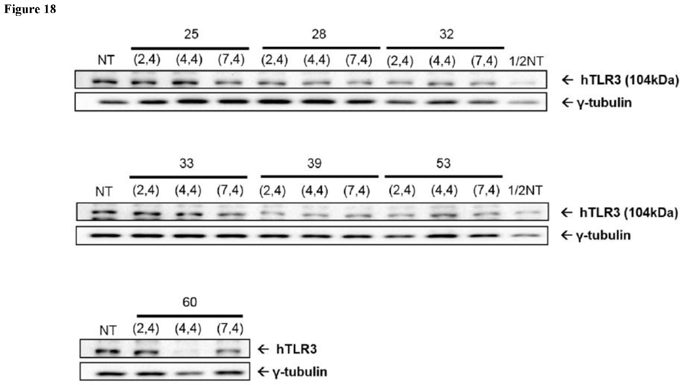

**Figure 18** shows the inhibition of TLR3 protein expression by exemplary cp-asiRNAs. The indicated cp-asiRNAs were contacted to HaCaT cells without transfection vehicle and, after 48 hours, protein was extracted and a western blot performed. (NT=no treatment).

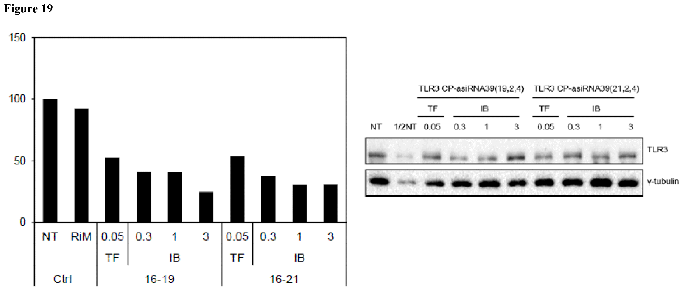

**Figure 19** shows the inhibition of TLR3 mRNA and protein expression by exemplary cp-asiRNAs. The indicated cp-asiRNAs were contacted to HaCaT cells without transfection vehicle and, after 48 hours, the degree of TLR3 expression was determined using qRT-PCR and Western blot.

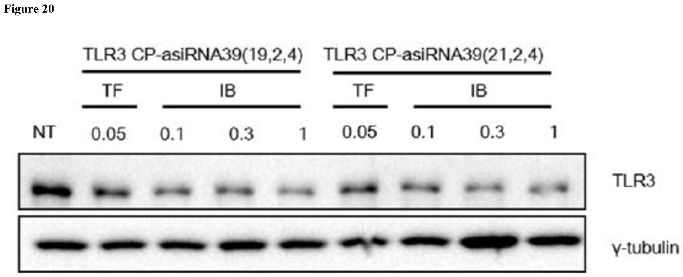

**Figure 20** shows the inhibition of TLR3 protein expression by exemplary cp-asiRNAs. The indicated cp-asiRNAs were contacted to HaCaT cells without transfection vehicle and, after 48 hours, protein was extracted and a western blot performed. (NT = no treatment).

### DETAILED DESCRIPTION

### General

In certain aspects, provided herein are asymmetric RNA complexes (*e.g.,* asiRNAs or lasiRNAs) that inhibit MyD88 and/or TLR3 expression and are therefore useful for the treatment of AMD (*e.g.,* wet AMD and/or dry AMD). In some embodiments, the RNA complexes are chemically modified to be capable of penetrating a cell without need for a transfection vehicle. In some embodiments, the RNA complex is an RNA complex listed in Table 1, Table 2, Table 3, Table 4, Table 5, Table 6, Table 8 or Table 10. In certain aspects, provided herein are pharmaceutical compositions comprising such RNA complexes and methods of using such RNA complexes and pharmaceutical compositions.

MyD88 is a protein that plays an important role in the onset of both dry AMD and wet AMD as one of the proteins that activate the immune response. Unlike previous AMD therapies that target VEGF, which are ineffective at treating dry macular degeneration, therapies targeting MyD88 can be used to treat both wet and dry AMD. An exemplary human MyD88 cDNA sequence is provided below.

### Human MyD88 cDNA sequence.

1 agattcctac ttcttacgcc ccccacatca cccgcctcga gacctcaagg gtagaggtgg
61 gcacccccgc ctccgcactt ttgctcgggg ctccagattg tagggcaggg cggcgcttct
121 cggaaagcga aagccggcgg ggcggggcgg gtgccgcagg agaaagagga agcgctggca
181 gacaatgcga cccgaccgcg ctgaggctcc aggaccgccc gccatggctg caggaggtcc
241 cggcgcgggg tctgcggccc cggtctcctc cacatcctcc cttcccctgg ctgctctcaa
301 catgcgagtg cggcgccgcc tgtctctgtt cttgaacgtg cggacacagg tggcggccga
361 ctggaccgcg ctggcggagg agatggactt tgagtacttg gagatccggc aactggagac
421 acaagcggac cccactggca ggctgctgga cgcctggcag ggacgccctg gcgcctctgt
481 aggccgactg ctcgagctgc ttaccaagct gggccgcgac gacgtgctgc tggagctggg
541 acccagcatt gaggaggatt gccaaaagta tatcttgaag cagcagcagg aggaggctga
601 gaagccttta caggtggccg ctgtagacag cagtgtccca cggacagcag agctggcggg
661 catcaccaca cttgatgacc ccctggggca tatgcctgag cgtttcgatg ccttcatctg
721 ctattgcccc agcgacatcc agtttgtgca ggagatgatc cggcaactgg aacagacaaa
781 ctatcgactg aagttgtgtg tgtctgaccg cgatgtcctg cctggcacct gtgtctggtc
841 tattgctagt gagctcatcg aaaagaggtg ccgccggatg gtggtggttg tctctgatga
901 ttacctgcag agcaaggaat gtgacttcca gaccaaattt gcactcagcc tctctccagg
961 tgcccatcag aagcgactga tccccatcaa gtacaaggca atgaagaaag agttccccag

1021 catcctgagg ttcatcactg tctgcgacta caccaacccc tgcaccaaat cttggttctg
1081 gactcgcctt gccaaggcct tgtccctgcc ctgaagactg ttctgaggcc ctgggtgtgt
1141 gtgtatctgt ctgcctgtcc atgtacttct gccctgcctc ctcctttcgt tgtaggagga
1201 atctgtgctc tacttacctc tcaattcctg gagatgccaa cttcacagac acgtctgcag
1261 cagctggaca tcacatttca tgtcctgcat ggaaccagtg gctgtgagtg gcatgtccac
1321 ttgctggatt atcagccagg acactataga acaggaccag ctgagactaa gaaggaccag
1381 cagagccagc tcagctctga gccattcaca catcttcacc ctcagtttcc tcacttgagg
1441 agtgggatgg ggagaacaga gagtagctgt gtttgaatcc ctgtaggaaa tggtgaagca
1501 tagctctggg tctcctgggg gagaccaggc ttggctgcgg gagagctggc tgttgctgga
1561 ctacatgctg gccactgctg tgaccacgac actgctgggg cagcttcttc cacagtgatg
1621 cctactgatg cttcagtgcc tctgcacacc gcccattcca cttcctcctt ccccacaggg
1681 caggtgggga agcagtttgg cccagcccaa ggagacccca ccttgagcct tatttcctaa
1741 tgggtccacc tctcatctgc atctttcaca cctcccagct tctgcccaac cttcagcagt
1801 gacaagtccc caagagactc gcctgagcag cttgggctgc ttttcatttc cacctgtcag
1861 gatgcctgtg gtcatgctct cagctccacc tggcatgaga agggatcctg gcctctggca
1921 tattcatcaa gtatgagttc tggggatgag tcactgtaat gatgtgagca gggagccttc
1981 ctccctgggc cacctgcaga gagctttccc accaactttg taccttgatt gccttacaaa
2041 gttatttgtt tacaaacagc gaccatataa aagcctcctg ccccaaagct tgtgggcaca
2101 tgggcacata cagactcaca tacagacaca cacatatatg tacagacatg tactctcaca
2161 cacacaggca ccagcataca cacgtttttc taggtacagc tcccaggaac agctaggtgg
2221 gaaagtccca tcactgaggg agcctaacca tgtccctgaa caaaaattgg gcactcatct
2281 attccttttc tcttgtgtcc ctactcattg aaaccaaact ctggaaagga cccaatgtac
2341 cagtatttat acctctaatg aagcacagag agaggaagag agctgcttaa actcacacaa
2401 caatgaactg cagacacagc tgttctctcc ctctctcctt cccagagcaa tttatacttt
2461 accctcaggc tgtcctctgg ggagaaggtg ccatggtctt aggtgtctgt gccccaggac
2521 agaccctagg accctaaatc caatagaaaa tgcatatctt tgctccactt tcagccaggc
2581 tggagcaagg taccttttct taggatcttg ggagggaatg gatgcccctc tctgcatgat
2641 cttgttgagg catttagctg ccatgcacct gtcccccttt aatactgggc attttaaagc
2701 catctcaaga ggcatcttct acatgttttg tacgcattaa aataatttca aagatatctg
2761 agaaaagccg atatttgcca ttcttcctat atcctggaat atatcttgca tcctgagttt
2821 ataataataa ataatattct accttggaaa aaaaaaaaaa aa

Toll-like receptor 3 (TLR3) plays a pivotal role in innate immune system as a type 1 transmembrane signaling molecule. TLR3 ligands include double-stranded RNA formed by the proliferation of RNA virus and polyinosinic-polycytidylic (polyI:C), a dsRNA analogue. In individuals suffering from dry AMD, alu-RNAs, a type of dsRNA, accumulate in retinal epithelial cells. Compared to a healthy individuals, people suffering from wet AMD had high expression of levels of TLR3 in peripheral blood mononuclear cells, indicating that TLR3 is closely associated in the pathogenesis of both dry and wet AMD. An exemplary human MyD88 cDNA sequence is provided below.

### Human TLR3 cDNA sequence.

1 cactttcgag agtgccgtct atttgccaca cacttccctg atgaaatgtc tggatttgga
61 ctaaagaaaa aaggaaaggc tagcagtcat ccaacagaat catgagacag actttgcctt
121 gtatctactt ttgggggggc cttttgccct ttgggatgct gtgtgcatcc tccaccacca
181 agtgcactgt tagccatgaa gttgctgact gcagccacct gaagttgact caggtacccg
241 atgatctacc cacaaacata acagtgttga accttaccca taatcaactc agaagattac
301 cagccgccaa cttcacaagg tatagccagc taactagctt ggatgtagga tttaacacca
361 tctcaaaact ggagccagaa ttgtgccaga aacttcccat gttaaaagtt ttgaacctcc
421 agcacaatga gctatctcaa ctttctgata aaacctttgc cttctgcacg aatttgactg
481 aactccatct catgtccaac tcaatccaga aaattaaaaa taatcccttt gtcaagcaga

541 agaatttaat cacattagat ctgtctcata atggcttgtc atctacaaaa ttaggaactc
601 aggttcagct ggaaaatctc caagagcttc tattatcaaa caataaaatt caagcgctaa
661 aaagtgaaga actggatatc tttgccaatt catctttaaa aaaattagag ttgtcatcga
721 atcaaattaa agagttttct ccagggtgtt ttcacgcaat tggaagatta tttggcctct
781 ttctgaacaa tgtccagctg ggtcccagcc ttacagagaa gctatgtttg gaattagcaa
841 acacaagcat tcggaatctg tctctgagta acagccagct gtccaccacc agcaatacaa
901 ctttcttggg actaaagtgg acaaatctca ctatgctcga tctttcctac aacaacttaa
961 atgtggttgg taacgattcc tttgcttggc ttccacaact agaatatttc ttcctagagt
1021 ataataatat acagcatttg ttttctcact ctttgcacgg gcttttcaat gtgaggtacc
1081 tgaatttgaa acggtctttt actaaacaaa gtatttccct tgcctcactc cccaagattg
1141 atgatttttc ttttcagtgg ctaaaatgtt tggagcacct taacatggaa gataatgata
1201 ttccaggcat aaaaagcaat atgttcacag gattgataaa cctgaaatac ttaagtctat
1261 ccaactcctt tacaagtttg cgaactttga caaatgaaac atttgtatca cttgctcatt
1321 ctcccttaca catactcaac ctaaccaaga ataaaatctc aaaaatagag agtgatgctt
1381 tctcttggtt gggccaccta gaagtacttg acctgggcct taatgaaatt gggcaagaac
1441 tcacaggcca ggaatggaga ggtctagaaa atattttcga aatctatctt tcctacaaca
1501 agtacctgca gctgactagg aactcctttg ccttggtccc aagccttcaa cgactgatgc
1561 tccgaagggt ggcccttaaa aatgtggata gctctccttc accattccag cctcttcgta
1621 acttgaccat tctggatcta agcaacaaca acatagccaa cataaatgat gacatgttgg
1681 agggtcttga gaaactagaa attctcgatt tgcagcataa caacttagca cggctctgga
1741 aacacgcaaa ccctggtggt cccatttatt tcctaaaggg tctgtctcac ctccacatcc
1801 ttaacttgga gtccaacggc tttgacgaga tcccagttga ggtcttcaag gatttatttg
1861 aactaaagat catcgattta ggattgaata atttaaacac acttccagca tctgtcttta
1921 ataatcaggt gtctctaaag tcattgaacc ttcagaagaa tctcataaca tccgttgaga
1981 agaaggtttt cgggccagct ttcaggaacc tgactgagtt agatatgcgc tttaatccct
2041 ttgattgcac gtgtgaaagt attgcctggt ttgttaattg gattaacgag acccatacca
2101 acatccctga gctgtcaagc cactaccttt gcaacactcc acctcactat catgggttcc
2161 cagtgagact tttgatacaa tcatcttgca aagacagtgc ccctttgaa ctcttttca
2221 tgatcaatac cagtatcctg ttgattttta tctttattgt acttctcatc cactttgagg
2281 gctggaggat atctttttat tggaatgttt cagtacatcg agttcttggt ttcaaagaaa
2341 tagacagaca gacagaacag tttgaatatg cagcatatat aattcatgcc tataaagata
2401 aggattgggt ctgggaacat ttctcttcaa tggaaaagga agaccaatct ctcaaatttt
2461 gtctggaaga aaggactttt gaggcgggtg tttttgaact agaagcaatt gttaacagca
2521 tcaaaagaag cagaaaaatt atttttgtta taacacacca tctattaaaa gacccattat
2581 gcaaaagatt caaggtacat catgcagttc aacaagctat tgaacaaaat ctggattcca
2641 ttatattggt tttccttgag gagattccag attataaact gaaccatgca ctctgtttgc
2701 gaagaggaat gtttaaatct cactgcatct tgaactggcc agttcagaaa gaacggatag
2761 gtgcctttcg tcataaattg caagtagcac ttggatccaa aaactctgta cattaaattt
2821 atttaaatat tcaattagca aaggagaaac tttctcaatt taaaaagttc tatggcaaat
2881 ttaagttttc cataaaggtg ttataatttg tttattcata tttgtaaatg attatattct
2941 atcacaatta catctcttct aggaaaatgt gtctccttat ttcaggccta tttttgacaa
3001 ttgacttaat tttacccaaa ataaaacata taagcacgta aaaaaaaaaa aaaaaaa

In some embodiments, the RNA complexes described herein are asiRNAs or lasiRNAs. As used herein, the term asiRNA refers to double-stranded asymmetrical short interfering RNA molecules that have a 19-21 nt antisense strand and a 13-17 nt sense strand. Additional information on asiRNAs can be found in U.S. Pat. Pub. No. 2012/0238017 and in Chang et al., Mol. Ther. 17:725-732 (2009), each of which is hereby incorporated by reference in its entirety. As used herein, the term lasiRNA refers to double-stranded long asymmetrical interfering RNA molecules that have a 13-21 nt sense strand and an antisense strand of greater than 24 nt. Additional information on lasiRNAs can be found in U.S. Pat. Pub. No. 2013/0273657, which is hereby incorporated by reference in its entirety.

In some embodiments, the RNA complexes described herein are delivered to cells using a delivery vehicle, such as liposomes, cationic polymers, cell penetrating peptides (CPPs), protein transduction domains (PTDs), antibodies and/or aptamers. In some embodiments, the RNA complex described herein is chemically modified so as to not require the use of such delivery vehicles to mediate MyD88 and/or TLR3 inhibition in a cell. Such RNA complexes are referred to herein as cell-penetrating asiRNAs (cp-asiRNAs) or cell-penetrating lasiRNAs (cp-lasiRNAs).

### Definitions

For convenience, certain terms employed in the specification, examples, and appended claims are collected here.

The articles "*a*" and "*an*" are used herein to refer to one or to more than one (*i.e.*, to at least one) of the grammatical object of the article. By way of example, "an element" means one element or more than one element.

As used herein, the term "*administering*" means providing a pharmaceutical agent or composition to a subject, and includes, but is not limited to, administering by a medical professional and self-administering.

As used herein, the terms "*interfering nucleic acid,*" "*inhibiting nucleic acid*" are used interchangeably. Interfering nucleic acids generally include a sequence of cyclic subunits, each bearing a base-pairing moiety, linked by intersubunit linkages that allow the base-pairing moieties to hybridize to a target sequence in a nucleic acid (typically an RNA) by Watson-Crick base pairing, to form a nucleic acid:oligomer heteroduplex within the target sequence. Interfering RNA molecules include, but are not limited to, antisense molecules, siRNA molecules, asiRNA molecules, lasiRNA molecules, single-stranded siRNA molecules, miRNA molecules and shRNA molecules. Such an interfering nucleic acids can be designed to block or inhibit translation of mRNA or to inhibit natural pre-mRNA splice processing, or induce degradation of targeted mRNAs, and may be said to be "directed to" or "targeted against" a target sequence with which it hybridizes. Interfering nucleic acids may include, for example, peptide nucleic acids (PNAs), locked nucleic acids (LNAs), 2'-O-Methyl oligonucleotides and RNA interference agents (siRNA agents). RNAi molecules generally act by forming a herteroduplex with the target molecule, which is selectively degraded or "knocked down," hence inactivating the target RNA. Under some conditions, an interfering RNA molecule can also inactivate a target transcript by repressing transcript translation and/or inhibiting transcription of the transcript. An interfering nucleic acid is more generally said to be "targeted against" a biologically relevant target, such as a protein, when it is targeted against the nucleic acid of the target in the manner described above.

The terms "*polynucleotide*", and "*nucleic acid*" are used interchangeably. They refer to a polymeric form of nucleotides, whether deoxyribonucleotides, ribonucleotides, or analogs thereof, in any combination and of any length. Polynucleotides may have any three-dimensional structure, and may perform any function. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. A polynucleotide may be further modified, such as by conjugation with a labeling component. In all nucleic acid sequences provided herein, U nucleotides are interchangeable with T nucleotides.

The phrase "*pharmaceutically-acceptable carrier*" as used herein means a pharmaceutically-acceptable material, composition or vehicle, such as a liquid or solid filler, diluent, excipient, or solvent encapsulating material.

An oligonucleotide "*specifically hybridizes*" to a target polynucleotide if the oligomer hybridizes to the target under physiological conditions, with a Tm substantially greater than 45° C, or at least 50° C, or at least 60° C-80° C or higher. Such hybridization corresponds to stringent hybridization conditions. At a given ionic strength and pH, the Tm is the temperature at which 50% of a target sequence hybridizes to a complementary polynucleotide. Again, such hybridization may occur with "near" or "substantial" complementarity of the antisense oligomer to the target sequence, as well as with exact complementarity.

As used herein, the term "*subject*" means a human or non-human animal selected for treatment or therapy.

The phrases "*therapeutically-effective amount*" and "*effective amount*" as used herein means the amount of an agent which is effective for producing the desired therapeutic effect in at least a sub-population of cells in a subject at a reasonable benefit/risk ratio applicable to any medical treatment.

"*Treating*" a disease in a subject or "*treating*" a subject having a disease refers to subjecting the subject to a pharmaceutical treatment, *e.g.*, the administration of a drug, such that at least one symptom of the disease is decreased or prevented from worsening.

As used herein, a therapeutic that "*prevents*" a disorder or condition refers to a compound that, when administered to a statistical sample prior to the onset of the disorder or condition, reduces the occurrence of the disorder or condition in the treated sample relative to an untreated control sample, or delays the onset or reduces the severity of one or more symptoms of the disorder or condition relative to the untreated control sample.

### RNA Complexes

In certain aspects, provided herein are RNA complexes that target MyD88 or TLR3 mRNA and inhibit MyD88 or TLR3 expression by a cell. The nucleic acid sequence of human MyD88 and TLR3 mRNA is provided in the sequence listing at the end of the disclosure.

In certain aspects, provided herein is an RNA complex comprising an antisense strand having sequence complementarity to a MyD88 or TLR3 mRNA sequence (*e.g.,* a human MyD88 or TLR3 mRNA sequence) and a sense strand having sequence complementarity to the antisense strand. In some embodiments, the RNA complex is capable of inhibiting MyD88 or TLR3 expression by a cell. In some embodiments, the RNA complex is an asymmetric short interfering RNA (an asiRNA). In some embodiments, the RNA complex is a long asymmetric short interfering RNA (a lasiRNA). In some embodiments, the RNA complex is an RNA complex listed in Table 1, Table 2, Table 3, Table 4, Table 5, Table 6, Table 8 or Table 10. The RNA complexes described herein can contain RNA bases, non-RNA bases or a mixture of RNA bases and non-RNA bases. For example, certain RNA complexes provided herein can be primarily composed of RNA bases but also contain DNA bases or non-naturally occurring nucleotides.

In some embodiments, the antisense strand is at least 19 nucleotides (nt) in length. In some embodiments, the antisense strand is 19 to 21 nt in length (*i.e.,* 19, 20 or 21 nt in length). In some embodiments, at least 13, 14, 15, 16, 17, 18, 19, 20 or 21 nt of the antisense strand are complementary to the MyD88 or TLR3 mRNA sequence. Perfect complementarity is not necessary. In some embodiments, the antisense strand is perfectly complementary to the MyD88 or TLR3 mRNA sequence.

In some embodiments, the antisense strand is at least 24 nt in length (*e.g.,* at least 25 nt in length, at least 26 nt in length, at least 27 nt in length, at least 28 nt in length, at least 29 nt in length, at least 30 nt in length or at least 31 nt in length). In some embodiments, the antisense strand is no greater than 124 nt in length (*e.g.,* no greater than 100 nt in length, no greater than 90 nt in length, no greater than 80 nt in length, no greater than 70 nt in length, no greater than 60 nt in length, no greater than 50 nt in length or no greater than 40 nt in length. In some embodiments, the antisense strand is 31 nt in length. In some embodiments, at least 16, 17, 18, 19, 20, 21, 22, 23, 24, 25, 26, 27, 29, 29, 30 or 31 nt of the antisense strand are complementary to the MyD88 or TLR3 mRNA sequence. Perfect complementarity is not necessary. In some embodiments, the antisense strand is perfectly complementary to the MyD88 or TLR3 mRNA sequence.

In some embodiments, the sense strand is 15 to 17 nt in length (*i.e.,* 15 nt in length, 16 nt in length or 17 nt in length). In some embodiments, at least 15 nt, at least 16 nt or at least 17 nt of the sense strand are complementary to the sequence of the antisense strand. In some embodiments the sense strand is perfectly complementary to the sequence of the antisense strand.

In some embodiments, the antisense strand and the sense strand form a complex in which the 5' end of the antisense strand and the 3' end of the sense strand form a blunt end. In some embodiments, the antisense strand and the sense strand form a complex in which the 5' end of the antisense strand overhangs the 3' end of the sense strand (*e.g.,* by 1, 2, 3, 4 or 5 nt). In some embodiments, the antisense strand and the sense strand form a complex in which the 5' end of the sense strand overhangs the 3' end of the antisense strand (*e.g.,* by 1, 2, 3, 4 or 5 nt).

In some embodiments, the antisense strand and/or the sense strand of the RNA complex has a sense strand sequence and/or an antisense strand sequence selected from the sequences listed in Table 1, Table 2, Table 3, Table 4, Table 5, Table 6, Table 8 or Table 10.

In some embodiments, the RNA complex provided herein comprises a chemical modification, wherein the modification facilitates the penetration of a cellular membrane in the absence of a delivery vehicle. In some embodiments, the modification is a 2'-O-methylated nucleoside, a phosphorothioate bond or a cholesterol moiety. In some embodiments, the RNA complex is a modified RNA complex listed in Table 2 or Table 4. In certain embodiments, the RNA complex is not cytotoxic.

The RNA complexes described herein can employ a variety of oligonucleotide chemistries. Examples of oligonucleotide chemistries include, without limitation, peptide nucleic acid (PNA), linked nucleic acid (LNA), phosphorothioate, 2'O-Me-modified oligonucleotides, and morpholino chemistries, including combinations of any of the foregoing. In general, PNA and LNA chemistries can utilize shorter targeting sequences because of their relatively high target binding strength relative to 2'O-Me oligonucleotides. Phosphorothioate and 2'O-Me-modified chemistries are often combined to generate 2'O-Me-modified oligonucleotides having a phosphorothioate backbone. See, *e.g.*, PCT Publication Nos. WO/2013/112053 and WO/2009/008725, each of which is hereby incorporated by reference in its entirety.

Peptide nucleic acids (PNAs) are analogs of DNA in which the backbone is structurally homomorphous with a deoxyribose backbone, consisting of N-(2-aminoethyl) glycine units to which pyrimidine or purine bases are attached. PNAs containing natural pyrimidine and purine bases hybridize to complementary oligonucleotides obeying Watson-Crick base-pairing rules, and mimic DNA in terms of base pair recognition. The backbone of PNAs is formed by peptide bonds rather than phosphodiester bonds, making them well-suited for antisense applications (see structure below). The backbone is uncharged, resulting in PNA/DNA or PNA/RNA duplexes that exhibit greater than normal thermal stability. PNAs are not recognized by nucleases or proteases.

Despite a radical structural change to the natural structure, PNAs are capable of sequence-specific binding in a helix form to DNA or RNA. Characteristics of PNAs include a high binding affinity to complementary DNA or RNA, a destabilizing effect caused by single-base mismatch, resistance to nucleases and proteases, hybridization with DNA or RNA independent of salt concentration and triplex formation with homopurine DNA. PANAGENE.TM. has developed its proprietary Bts PNA monomers (Bts; benzothiazole-2-sulfonyl group) and proprietary oligomerization process. The PNA oligomerization using Bts PNA monomers is composed of repetitive cycles of deprotection, coupling and capping. PNAs can be produced synthetically using any technique known in the art. See, *e.g.*, U.S. Pat. Nos. 6,969,766, 7,211,668, 7,022,851, 7,125,994, 7,145,006 and 7,179,896. See also U.S. Pat. Nos. 5,539,082; 5,714,331; and 5,719,262 for the preparation of PNAs. Further teaching of PNA compounds can be found in Nielsen et al., Science, 254:1497-1500, 1991. Each of the foregoing is incorporated by reference in its entirety.

Interfering nucleic acids may also contain "locked nucleic acid" subunits (LNAs). "LNAs" are a member of a class of modifications called bridged nucleic acid (BNA). BNA is characterized by a covalent linkage that locks the conformation of the ribose ring in a C3-endo (northern) sugar pucker. For LNA, the bridge is composed of a methylene between the 2'-O and the 4'-C positions. LNA enhances backbone preorganization and base stacking to increase hybridization and thermal stability.

The structures of LNAs can be found, for example, in Wengel, et al., Chemical Communications (1998) 455; Tetrahedron (1998) 54:3607, and Accounts of Chem. Research (1999) 32:301); Obika, et al., Tetrahedron Letters (1997) 38:8735; (1998) 39:5401, and Bioorganic Medicinal Chemistry (2008) 16:9230. Compounds provided herein may incorporate one or more LNAs; in some cases, the compounds may be entirely composed of LNAs. Methods for the synthesis of individual LNA nucleoside subunits and their incorporation into oligonucleotides are described, for example, in U.S. Pat. Nos. 7,572,582, 7,569,575, 7,084,125, 7,060,809, 7,053,207, 7,034,133, 6,794,499, and 6,670,461, each of which is incorporated by reference in its entirety. Typical intersubunit linkers include phosphodiester and phosphorothioate moieties; alternatively, non-phosphorous containing linkers may be employed. One embodiment is an LNA-containing compound where each LNA subunit is separated by a DNA subunit. Certain compounds are composed of alternating LNA and DNA subunits where the intersubunit linker is phosphorothioate.

In certain embodiments, the RNA complex is linked to a cholesterol moiety. In some embodiments, the cholesterol moiety is attached to the 3' terminus of the sense strand. In some embodiments, the cholesterol moiety is attached to the 3' terminus of the antisense strand. In some embodiments, the cholesterol moiety is attached to the 5' terminus of the sense strand. In some embodiments, the cholesterol moiety is attached to the 5' terminus of the antisense strand.

In some embodiments, the RNA complex comprises a 2'-O-methylated nucleoside. 2'-O-methylated nucleosides carry a methyl group at the 2'-OH residue of the ribose molecule. 2'-O-Me-RNAs show the same (or similar) behavior as RNA, but are protected against nuclease degradation. 2'-O-Me-RNAs can also be combined with phosphothioate oligonucleotides (PTOs) for further stabilization. 2'-O-Me-RNAs (phosphodiester or phosphothioate) can be synthesized according to routine techniques in the art (see, *e.g.*, Yoo et al., Nucleic Acids Res. 32:2008-16, 2004, which is hereby incorporated by reference).

In some embodiments, the 2'-O-methyl nucleoside is positioned at the 3' terminus of the sense strand. In some embodiments, 3' terminal region of the sense strand comprises a plurality of 2'-O-methylated nucleosides (*e.g.,* 2, 3, 4, 5 or 6 2'-O-methylated nucleosides within 6 nucleosides of the 3' terminus). In some embodiments, the 2'-O-methyl nucleoside is positioned at the 3' terminus of the antisense strand. In some embodiments, 3' terminal region of the antisense strand comprises a plurality of 2'-O-methylated nucleosides (*e.g.,* 2, 3, 4, 5 or 6 2'-O-methylated nucleosides within 6 nucleosides of the 3' terminus). In some embodiments, both the 3' terminal region of the sense strand and the 3' terminal region of the antisense strand comprise a plurality of 2'-O-methylated nucleosides. In some embodiments, the sense strand comprises 2'-O-methylated nucleosides that alternate with unmodified nucleosides. In some embodiments, the sense strand comprises a contiguous sequence of 2, 3, 4, 5, 6, 7 or 8 2'-O-methylated nucleosides that alternate with unmodified nucleosides. In some embodiments, the anti-sense strand comprises 2'-O-methylated nucleosides that alternate with unmodified nucleosides. In some embodiments, the anti-sense strand comprises a contiguous sequence of 2, 3, 4, 5, 6, 7 or 8 2'-O-methylated nucleosides that alternate with unmodified nucleosides.

In some embodiments, the RNA complex comprises a phosphorothioate bond. "Phosphorothioates" (or S-oligos) are a variant of normal DNA in which one of the nonbridging oxygens is replaced by a sulfur. The sulfurization of the internucleotide bond reduces the action of endo-and exonucleases including 5' to 3' and 3' to 5' DNA POL 1 exonuclease, nucleases S1 and P1, RNases, serum nucleases and snake venom phosphodiesterase. Phosphorothioates are made by two principal routes: by the action of a solution of elemental sulfur in carbon disulfide on a hydrogen phosphonate, or by the method of sulfurizing phosphite triesters with either tetraethylthiuram disulfide (TETD) or 3H-1,2-benzodithiol-3-one 1,1-dioxide (BDTD) (see, *e.g.*, Iyer et al., J. Org. Chem. 55, 4693-4699, 1990). The latter methods avoid the problem of elemental sulfur's insolubility in most organic solvents and the toxicity of carbon disulfide. The TETD and BDTD methods also yield higher purity phosphorothioates.

In some embodiments, at least 25%, 30%, 35%, 40%, 45%, 50%, 55%, 60%, 65%, 70%, 75%, 80%, 85%, 90% or 95% of the bonds between the ribonucleotides in the sense strand of the RNA complex are phosphorothioate bonds. In some embodiments, all of the bonds between the ribonucleotides in the sense strand of the RNA complex are phosphorothioate bonds.

In some embodiments, at least 25%, 30%, 35%, 40%, 45%, 50%, 55%, 60%, 65%, 70%, 75%, 80%, 85%, 90% or 95% of the bonds between the ribonucleotides in the antisense strand of the RNA complex are phosphorothioate bonds. In some embodiments, all of the bonds between the ribonucleotides in the antisense strand of the RNA complex are phosphorothioate bonds.

The RNA complexes described herein may be contacted with a cell or administered to an organism (*e.g.,* a human). Alternatively, constructs and/or vectors encoding the RNA complexes may be contacted with or introduced into a cell or organism. In certain embodiments, a viral, retroviral or lentiviral vector is used.

The RNA complexes described herein can be prepared by any appropriate method known in the art. For example, in some embodiments, the RNA complexes described herein are prepared by chemical synthesis or in vitro transcription.

In certain aspects, provided herein is a pharmaceutical composition comprising an RNA complex provided herein and a pharmaceutically acceptable carrier. In certain embodiments, the pharmaceutical composition is formulated for delivery to the eye (*e.g.,* as an eye drop). In some embodiments, the pharmaceutical composition is formulated for intravitreal delivery.

In some embodiments, the pharmaceutical composition further comprises a second agent for treatment of AMD (*e.g.,* an anti-VEGF therapeutic, such as bevacizumab, ranibizumab, pegaptanib and/or aflibercept). In certain embodiments, the pharmaceutical composition does not comprise a transfection vehicle. In some embodiments, the pharmaceutical composition comprises a delivery vehicle (*e.g.,* liposomes, cationic polymers, cell penetrating peptides (CPPs), protein transduction domains (PTDs), antibodies and/or aptamers). In some embodiments, the composition includes a combination of multiple (*e.g.,* two or more) of the RNA complexes described herein.

Methods of preparing these formulations or compositions include the step of bringing into association an RNA complex described herein with the carrier and, optionally, one or more accessory ingredients. In general, the formulations are prepared by uniformly and intimately bringing into association an agent described herein with liquid carriers.

### Therapeutic Methods

In certain aspects, provided herein is a method of inhibiting MyD88 and/or TLR3 expression by a cell comprising contacting the cell with an RNA complex provided herein. In some embodiments, the RNA complex is a modified RNA complex and the cell is contacted with the RNA complex in the absence of a transfection vehicle. In some embodiments, the cell is contacted with the RNA complex in the presence of a delivery vehicle (*e.g.,* a liposome, cationic polymer, cell penetrating peptide (CPPs), protein transduction domain (PTDs), antibody and/or aptamer). In some embodiments, the cell is present in the eye of a human subject. In some embodiments, the subject has AMD (*e.g.,* wet AMD or dry AMD).

In certain aspects, provided herein is a method of treating a human subject for AMD (*e.g.,* wet AMD or dry AMD) comprising administering to the subject an RNA complex or pharmaceutical composition provided herein. In certain embodiments, the RNA complex or pharmaceutical composition is administered to the eye of the subject. In some embodiments, the RNA complex or pharmaceutical composition self-administered by the subject.

In the present methods, an RNA complex described herein can be administered to the subject, for example, as nucleic acid without delivery vehicle (*e.g.,* for cp-asiRNAs and cp-lasiRNAs), in combination with a delivery reagent, and/or as a nucleic acid comprising sequences that express the RNA complex described herein. In some embodiments, any nucleic acid delivery method known in the art can be used in the methods described herein. Suitable delivery reagents include, but are not limited to, *e.g.,* the Mirus Transit TKO lipophilic reagent; lipofectin; lipofectamine; cellfectin; polycations (*e.g.,* polylysine), atelocollagen, nanoplexes and liposomes. The use of atelocollagen as a delivery vehicle for nucleic acid molecules is described in Minakuchi et al. Nucleic Acids Res., 32(13):e109

(2004); Hanai et al. Ann NY Acad Sci., 1082:9-17 (2006); and Kawata et al. Mol Cancer Ther., 7(9):2904-12 (2008); each of which is incorporated herein in their entirety. Exemplary interfering nucleic acid delivery systems are provided in U.S. Patent Nos. 8,283,461, 8,313,772, 8,501,930. 8,426,554, 8,268,798 and 8,324,366, each of which is hereby incorporated by reference in its entirety.

In some embodiments of the methods described herein, liposomes are used to deliver an RNA complex described herein to a subject. Liposomes suitable for use in the methods described herein can be formed from standard vesicle-forming lipids, which generally include neutral or negatively charged phospholipids and a sterol, such as cholesterol. The selection of lipids is generally guided by consideration of factors such as the desired liposome size and half-life of the liposomes in the blood stream. A variety of methods are known for preparing liposomes, for example, as described in Szoka et al. (1980), Ann. Rev. Biophys. Bioeng. 9:467; and U.S. Pat. Nos. 4,235,871, 4,501,728, 4,837,028, and 5,019,369, the entire disclosures of which are herein incorporated by reference.

The liposomes for use in the present methods can also be modified so as to avoid clearance by the mononuclear macrophage system ("MMS") and reticuloendothelial system ("RES"). Such modified liposomes have opsonization-inhibition moieties on the surface or incorporated into the liposome structure.

Opsonization-inhibiting moieties for use in preparing the liposomes described herein are typically large hydrophilic polymers that are bound to the liposome membrane. As used herein, an opsonization inhibiting moiety is "bound" to a liposome membrane when it is chemically or physically attached to the membrane, *e.g.,* by the intercalation of a lipid-soluble anchor into the membrane itself, or by binding directly to active groups of membrane lipids. These opsonization-inhibiting hydrophilic polymers form a protective surface layer that significantly decreases the uptake of the liposomes by the MMS and RES; *e.g.,* as described in U.S. Pat. No. 4,920,016, the entire disclosure of which is herein incorporated by reference.

In some embodiments, opsonization inhibiting moieties suitable for modifying liposomes are water-soluble polymers with a number-average molecular weight from about 500 to about 40,000 daltons, or from about 2,000 to about 20,000 daltons. Such polymers include polyethylene glycol (PEG) or polypropylene glycol (PPG) derivatives; *e.g.,* methoxy PEG or PPG, and PEG or PPG stearate; synthetic polymers such as polyacrylamide or poly N-vinyl pyrrolidone; linear, branched, or dendrimeric polyamidoamines; polyacrylic acids; polyalcohols, *e.g.,* polyvinylalcohol and polyxylitol to which carboxylic or amino groups are chemically linked, as well as gangliosides, such as ganglioside GM1. Copolymers of PEG, methoxy PEG, or methoxy PPG, or derivatives thereof, are also suitable. In addition, the opsonization inhibiting polymer can be a block copolymer of PEG and either a polyamino acid, polysaccharide, polyamidoamine, polyethyleneamine, or polynucleotide. The opsonization inhibiting polymers can also be natural polysaccharides containing amino acids or carboxylic acids, *e.g.,* galacturonic acid, glucuronic acid, mannuronic acid, hyaluronic acid, pectic acid, neuraminic acid, alginic acid, carrageenan; aminated polysaccharides or oligosaccharides (linear or branched); or carboxylated polysaccharides or oligosaccharides, *e.g.,* reacted with derivatives of carbonic acids with resultant linking of carboxylic groups. In some embodiments, the opsonization-inhibiting moiety is a PEG, PPG, or derivatives thereof. Liposomes modified with PEG or PEG-derivatives are sometimes called "PEGylated liposomes."

The pharmaceutical compositions disclosed herein may be delivered by any suitable route of administration, including topically, intravitreally, orally and parenterally. In certain embodiments the pharmaceutical compositions are delivered generally (*e.g.,* via oral or parenteral administration). In certain other embodiments the pharmaceutical compositions are delivered locally through direct administration to the eye.

Actual dosage levels of the RNA complexes in the pharmaceutical compositions may be varied so as to obtain an amount of RNA complex that is effective to achieve the desired therapeutic response for a particular patient, composition, and mode of administration, without being toxic to the patient.

The selected dosage level will depend upon a variety of factors including the activity of the particular agent employed, the route of administration, the time of administration, the rate of excretion or metabolism of the particular compound being employed, the duration of the treatment, other drugs, compounds and/or materials used in combination with the particular compound employed, the age, sex, weight, condition, general health and prior medical history of the patient being treated, and like factors well known in the medical arts.

A physician having ordinary skill in the art can readily determine and prescribe the effective amount of the pharmaceutical composition required. For example, the physician or veterinarian could prescribe and/or administer doses of the agents employed in the pharmaceutical composition at levels lower than that required in order to achieve the desired therapeutic effect and gradually increase the dosage until the desired effect is achieved.

In general, a suitable daily dose of an RNA complex described herein will be that amount of the RNA complex which is the lowest dose effective to produce a therapeutic effect. Such an effective dose will generally depend upon the factors described above.

### EXEMPLIFICATION

### Example 1: Screening for MyD88-specific asymmetric small interfering RNAs

To identify asymmetric small interfering RNAs (asiRNAs) that inhibit MyD88 with high efficiency, 44 asiRNAs were synthesized and screened. The nucleic acid sequences of the screened asiRNAs are provided in Table 1.

**Table 1: Nucleic acid sequences for exemplary MyD88-targeting asiRNA.**

| **asiRNA SEQUENCE** |
|---|
| MyD88#1(S) : 5' GGCGGCCGACUGGACC 3' |
| MyD88#1(AS) : 5' GGUCCAGUCGGCCGCCACC 3' |
| MyD88#2(S) : 5' UGGCGGCCGACUGGAC 3' |
| MyD88#2(AS) : 5' GUCCAGUCGGCCGCCACCU 3' |
| MyD88#3(S) : 5' GUGGCGGCCGACUGGA 3' |
| MyD88#3(AS) : 5' UCCAGUCGGCCGCCACCUG 3' |
| MyD88#4(S) : 5' CUGGCGGAGGAGAUGG 3' |
| MyD88#4(AS) : 5' CCAUCUCCUCCGCCAGCGC 3' |
| MyD88#5(S) : 5' GCUGGCGGAGGAGAUG 3' |
| MyD88#5(AS) : 5' CAUCUCCUCCGCCAGCGCG 3' |
| MyD88#6(S) : 5' AGUACUUGGAGAUCCG 3' |
| MyD88#6(AS) : 5' CGGAUCUCCAAGUACUCAA 3' |
| MyD88#7(S) : 5' GAGUACUUGGAGAUCC 3' |
| MyD88#7(AS) : 5' GGAUCUCCAAGUACUCAAA 3' |
| MyD88#8(S) : 5' GCCUUUACAGGUGGCC 3' |
| MyD88#8(AS) : 5' GGCCACCUGUAAAGGCUUC 3' |
| MyD88#9(S) : 5' AGCCUUUACAGGUGGC 3' |
| MyD88#9(AS) : 5' GCCACCUGUAAAGGCUUCU 3' |
| MyD88#10(S) : 5' AAGCCUUUACAGGUGG 3' |
| MyD88#10(AS) : 5' CCACCUGUAAAGGCUUCUC 3' |
| MyD88#11(S) : 5' GAAGCCUUUACAGGUG 3' |
| MyD88#11(AS) : 5' CACCUGUAAAGGCUUCUCA 3' |
| MyD88#12(S) : 5' AGAAGCCUUUACAGGU3' |
| MyD88#12(AS) : 5' ACCUGUAAAGGCUUCUCAG 3' |
| MyD88#13(S) : 5' AGAUGAUCCGGCAACU 3' |
| MyD88#13(AS) : 5' AGUUGCCGGAUCAUCUCCU 3' |
| MyD88#14(S) : 5' GAGAUGAUCCGGCAAC 3' |
| MyD88#14(AS) : 5' GUUGCCGGAUCAUCUCCUG 3' |
| MyD88#15(S) : 5' GGAGAUGAUCCGGCAA 3' |
| MyD88#15(AS) : 5' UUGCCGGAUCAUCUCCUGC 3' |
| MyD88#16(S) : 5' AGGAGAUGAUCCGGCA 3' |
| MyD88#16(AS) : 5' UGCCGGAUCAUCUCCUGCA 3' |
| MyD88#17(S) : 5' CAGGAGAUGAUCCGGC 3' |
| MyD88#17(AS) : 5' GCCGGAUCAUCUCCUGCAC 3' |
| MyD88#18(S) : 5' GCAGGAGAUGAUCCGG 3' |
| MyD88#18(AS) : 5' CCGGAUCAUCUCCUGCACA 3' |
| MyD88#19(S) : 5' UGCAGGAGAUGAUCCG 3' |
| MyD88#19(AS) : 5' CGGAUCAUCUCCUGCACAA 3' |
| MyD88#20(S) : 5' GUGCAGGAGAUGAUCC 3' |
| MyD88#20(AS) : 5' GGAUCAUCUCCUGCACAAA 3' |
| MyD88#21(S) : 5' UGUGCAGGAGAUGAUC 3' |
| MyD88#21(AS) : 5' GAUCAUCUCCUGCACAAAC 3' |
| MyD88#22(S) : 5' UUGUGCAGGAGAUGAU 3' |
| MyD88#22(AS) : 5' AUCAUCUCCUGCACAAACU 3' |
| MyD88#23(S) : 5' UUUGUGCAGGAGAUGA 3' |
| MyD88#23(AS) : 5' UCAUCUCCUGCACAAACUG 3' |
| MyD88#24(S) : 5' GUUUGUGCAGGAGAUG 3' |
| MyD88#24(AS) : 5' CAUCUCCUGCACAAACUGG 3' |
| MyD88#25(S) : 5' AGUUUGUGCAGGAGAU 3' |
| MyD88#25(AS) : 5' AUCUCCUGCACAAACUGGA 3' |
| MyD88#26(S) : 5' GUGACUUCCAGACCAA 3' |
| MyD88#26(AS) : 5' UUGGUCUGGAAGUCACAUU 3' |
| MyD88#27(S) : 5' UGUGACUUCCAGACA 3' |
| MyD88#27(AS) : 5' UGGUCUGGAAGUCACAUUC 3' |
| MyD88#28(S) : 5' AUGUGACUUCCAGACC 3' |
| MyD88#28(AS) : 5' GGUCUGGAAGUCACAUUCC 3' |
| MyD88#29(S) : 5' AAUGUGACUUCCAGAC 3' |
| MyD88#29(AS) : 5' GUCUGGAAGUCACAUUCCU 3' |
| MyD88#30(S) : 5' GAAUGUGACUUCCAGA 3' |
| MyD88#30(AS) : 5' UCUGGAAGUCACAUUCCUU 3' |
| MyD88#31(S) : 5' GGAAUGUGACUUCCAG 3' |
| MyD88#31(AS) : 5' CUGGAAGUCACAUUCCUUG 3' |
| MyD88#32(S) : 5' AGGAAUGUGACUUCCA 3' |
| MyD88#32(AS) : 5' UGGAAGUCACAUUCCUUGC 3' |
| MyD88#33(S) : 5' AAGGAAUGUGACUUCC 3' |
| MyD88#33(AS) : 5' GGAAGUCACAUUCCUUGCU 3' |
| MyD88#34(S) : 5' CAAGGAAUGUGACUUC 3' |
| MyD88#34(AS) : 5' GAAGUCACAUUCCUUGCUC 3' |
| MyD88#35(S) : 5' GCAAGGAAUGUGACUU 3' |
| MyD88#35(AS) : 5' AAGUCACAUUCCUUGCUCU 3' |
| MyD88#36(S) : 5' AGCAAGGAAUGUGACU 3' |
| MyD88#36(AS) : 5' AGUCACAUUCCUUGCUCUG 3' |
| MyD88#37(S) : 5' GAGCAAGGAAUGUGAC 3' |
| MyD88#37(AS) : 5' GUCACAUUCCUUGCUCUGC 3' |
| MyD88#38(S) : 5' AGAGCAAGGAAUGUGA 3' |
| MyD88#38(AS) : 5' UCACAUUCCUUGCUCUGCA 3' |
| MyD88#39(S) : 5' CAGAGCAAGGAAUGUG 3' |
| MyD88#39(AS) : 5' CACAUUCCUUGCUCUGCAG 3' |
| MyD88#40(S) : 5' GUCCCUGCCCUGAAGA 3' |
| MyD88#40(AS) : 5' UCUUCAGGGCAGGGACAAG 3' |
| MyD88#41(S) : 5' UGUCCCUGCCCUGAAG 3' |
| MyD88#41(AS) : 5' CUUCAGGGCAGGGACAAGG 3' |
| MyD88#42(S) : 5' UUGUCCCUGCCCUGAA 3' |
| MyD88#42(AS) : 5' UUCAGGGCAGGGACAAGGC 3' |
| MyD88#43(S) : 5' GCACCUGUGUCUGGUC 3' |
| MyD88#43(AS) : 5' GACCAGACACAGGUGCCAG 3' |
| MyD88#44(S) : 5' GGCACCUGUGUCUGGU 3' |
| MyD88#44(AS) : 5' ACCAGACACAGGUGCCAGG 3' |

The asiRNAs listed in Table 1 were incubated at 95 °C for 2 minutes and at 37 °C for 1 hour in 1x siRNA duplex buffer (STpharm). Proper strand annealing was confirmed via gel electrophoresis. For the screen, HeLa cells (ATCC) were used that had been cultured in Dulbecco's modified Eagle's medium (Gibco) containing 10% fetal bovine serum (Gibco) and 100 µg/ml penicillin/streptomycin in a 100 mm cell culture dish. One day prior to transfection, 2.0 x 10⁴ HeLa cells were seeded in 24-well plates. The HeLa cells were transfected with 0.3 nM of the asiRNAs using Lipofectamine 2000 (Invitrogen) according to the manufacturer's instructions.

The MyD88 mRNA levels in the transfected cells were measured 24 hours after transfection using real-time PCR. Specifically, total RNA were extracted using RNAiso Plus (TaKaRa), and then 500 ng of the extracted RNA was used for cDNA synthesis using the High-capacity cDNA reverse transcription kit (Applied Biosystems), according to the manufacturer's instructions. The synthesized cDNA was diluted and then quantitative real-time PCR was performed using the StepOne real-time PCR system (Applied Biosystems) according to manufacturer's instructions. Amplification of the MyD88 gene was detected using a power SYBR Premix Ex Taq (TaKaRa). GAPDH was amplified as an internal control. The following primer sequences were used:
*Human GAPDH*-forward 5'-GAG TCA ACG GAT TTG GTC GT-3'
*Human GAPDH*-reverse 5'-GAC AAG CTT CCC GTT CTC AG-3'
*Human MyD88-*forward 5'- AAG TTA TTT GTT TAC AAA CAG CGA CCA -3'
*Human MyD88-*reverse 5'- GGA AGA ATG GCA AAT ATC GGC T -3'

The level of MyD88 inhibition by each of the 44 asiRNAs is provided in Figure 1. Three of the asiRNA sequences, asiMyD88(26), asiMyD88(27) and asiMyD88(32), were selected for use in follow-up studies.

### Example 2: Inhibition of MyD88 mRNA expression level using MyD88-targeting asiRNAs

Three of the asiRNA sequences, asiMyD88(26), asiMyD88(27) and asiMyD88(32), were tested for their ability to inhibit MyD88 expression at different concentrations. The asiRNAs were incubated at 95 °C for 2 minutes and at 37 °C for 1 hour in 1x siRNA duplex buffer (STpharm). Proper strand annealing was confirmed via gel electrophoresis. For the screen, HeLa cells (ATCC) were used that had been cultured in Dulbecco's modified Eagle's medium (Gibco) containing 10% fetal bovine serum (Gibco) and 100 µg/ml penicillin/streptomycin in a 100 mm cell culture dish. One day prior to transfection, 2.0 x 10⁴ HeLa cells were seeded in 24-well plates. The HeLa cells were transfected with asiRNAs using Lipofectamine 2000 (Invitrogen) according to the manufacturer's instructions.

The level of MyD88 inhibition by the different concentrations of the 3 asiRNAs is provided in Figure 2. As seen in Figure 2, low concentration of asiMyD88(26) and asiMyD88(27) exhibited the highest levels of MyD88 inhibition. asiMyD88(26) and asiMyD88(27) were selected for use in follow-up studies.

### Example 3: Modification of asiRNAs

A variety of potential asiMyD88 structures having different antisense strand lengths were synthesized and tested for their ability to inhibit MyD88 expression. (Table 2)

**Table 2. Additional asiRNA sequences.**

| |
|---|
| MyD88#26(S) : 5' GUGACUUCCAGACCAA 3' |
| MyD88#26(19AS) : 5' UUGGUCUGGAAGUCACAUU 3' |
| MyD88#26(21AS) : 5' UUGGUCUGGAAGUCACAUUCC 3' |
| MyD88#26(31AS) : 5' UUGGUCUGGAAGUCACAUUCCUUGCUCUGCA 3' |
| MyD88#27(S) : 5' UGUGACUUCCAGCCA 3' |
| MyD88#27(19AS) : 5' UGGUCUGGAAGUCACAUUC 3' |
| MyD88#27(21AS) : 5' UGGUCUGGAAGUCACAUUCCU 3' |
| MyD88#27(31AS) : 5' UGGUCUGGAAGUCACAUUCCUUGCUCUGCAG 3' |

The asiRNAs listed in Table 2 were incubated at 95 °C for 2 minutes and at 37 °C for 1 hour in 1x siRNA duplex buffer (STpharm). Proper strand annealing was confirmed via gel electrophoresis. For the screen, HeLa cells (ATCC) were used that had been cultured in Dulbecco's modified Eagle's medium (Gibco) containing 10% fetal bovine serum (Gibco) and 100 µg/ml penicillin/streptomycin in a 100 mm cell culture dish. One day prior to transfection, 2.0 x 10⁴ HeLa cells were seeded in 24-well plates. The HeLa cells were transfected with 0.3 nM of the asiRNAs using Lipofectamine 2000 (Invitrogen) according to the manufacturer's instructions.

The MyD88 mRNA levels in the transfected cells were measured 24 hours after transfection using real-time RT-PCR. Specifically, total RNA were extracted using RNAiso Plus(TaKaRa), and then 500 ng of the extracted RNA was used for cDNA synthesis using the High-capacity cDNA reverse transcription kit (Applied Biosystems), according to the manufacturer's instructions. The synthesized cDNA was diluted and then quantitative real-time PCR was performed using the StepOne real-time PCR system (Applied Biosystems) according to manufacturer's instructions. Amplification of the MyD88 gene was detected using a power SYBR Premix Ex Taq (TaKaRa). GAPDH was amplified as an internal control.

The level of MyD88 inhibition by each of the 6 asiRNAs is provided in Figure 3. 21 nucleotide antisense of asiMyD88(26) and asiMyD88(27) exhibited the highest levels of MyD88 inhibition. 21 nucleotide antisense were selected for use in follow-up studies.

### Example 4: Inhibition of MyD88 protein using MyD88-specific asiRNAs

The efficacy of asiMyD88 for the inhibition of MyD88 protein was tested.

The asiRNAs were incubated at 95 °C for 2 minutes and at 37 °C for 1 hour in 1x siRNA duplex buffer (STpharm). Proper strand annealing was confirmed via gel electrophoresis.

A549 cells (ATCC) and HeLa cells (ATCC) were used that had been cultured in Dulbecco's modified Eagle's medium (Gibco) containing 10% fetal bovine serum (Gibco) and 100 µg/ml penicillin/streptomycin in a 100 mm cell culture dish. One day prior to transfection, 5.0 x 10⁴ A549 cells or HeLa cells were seeded in 12-well plates. A549cells and HeLa cells were transfected with 10 nM and 3 nM of the asiRNAs using Lipofectamine^{TM} RNAiMAX (Invitrogen) according to the manufacturer's instructions. After 24 hours, OPTI-MEM media was replaced with a serum-containing media.

After 48 hours of asiRNA transfection, the level of MyD88 protein expression was determined via western blot. Briefly, the transfected A549 cells and HeLa cells were lysed with RIPA buffer (GE). 15 µg of the total protein extract of A549 cells or 30 µg of the total protein extract of HeLa cells were loaded onto a 12% SDS-PAGE gel and electrophoresed at 120 V. After electrophoresis, the proteins were transferred to PVDF membrane (Bio-rad) already activated by methanol (Merck) for 1 hour at 300 mA. The membrane was blocked for 1 hour at the room temperature with 5% skim milk (Seoul Milk) and then incubated overnight at 4°C in 5% skim milk containing anti-MyD88 antibody (Abcam) and anti-β-actin antibody (Santa Cruz). The membrane was then washed with 1x TBST for 10 minutes three times and was incubated for 1 hour at the room temperature in 5% skim milk with HRP-conjugated secondary antibody. The membrane was washed with 1x TBST for 10 minutes and treated with 1x ECL for 1 minute. The MyD88 and β-actin bands were then imaged using a Chemidoc instrument (Bio-rad).

The results of the western blot assay are depicted in Figure 4. In all asiMyD88 transfection cell lines of A549 cells and HeLa cells, 80% or more of MyD88 protein inhibition were confirmed. (Figure 4).

### Example 5: Chemical modification of MyD88 asiRNAs

Chemical modifications were applied to the asiRNAs. As described below, certain of the modifications improved endocytosis and stability of the asiRNAs. Four asiRNAs (Table 3) were tested for MyD88 mRNA inhibition in HeLa cells

**Table 3. Modified asiRNA sequences. m = 2'-O-Methyl RNA**

| |
|---|
| MyD88#26(16S-1) : 5' mGUmGAmCUmUCmCAmGAmCCmAA 3' |
| MyD88#26(19AS-1) : 5' UUGGUCUGGAAGUCmAmCmAmUmU 3' |
| MyD88#26(21AS-1) : 5' UUGGUCUGGAAGUCmAmCmAmUmUmCmC 3' |
| MyD88#27(16S-1) : 5' mUGmUGmACmUUmCCmAGmACmCA 3' |
| MyD88#27(19AS-1) : 5' UGGUCUGGAAGUCAmCmAmUmUmC 3' |
| MyD88#27(21AS-1) : 5' UGGUCUGGAAGUCAmCmAmUmUmCmCmU 3' |

HeLa cells (ATCC) were used that had been cultured in Dulbecco's modified Eagle's medium (Gibco) containing 10% fetal bovine serum (Gibco) and 100 µg/ml penicillin/streptomycin in a 100 mm cell culture dish. One day prior to transfection, 2.0 x 10⁴ HeLa cells were seeded in 24-well plates. The HeLa cells were transfected using Lipofectamine 2000 (Invitrogen) according to the manufacturer's instructions. Twenty-four hours later, MyD88 mRNA levels were in the HeLa cells were determined.

The level of MyD88 inhibition by each of the asiRNAs in provided in Figure 5. Modified MyD88(27) exhibited the highest levels of MyD88 inhibition.

### Example 6: Chemical modification of asiRNAs for self-delivery

Chemical modifications were applied to the asiRNAs and the cellular delivery of the modified asiRNAs was tested in the absence of other delivery vehicle. As described below, certain of the modifications improved endocytosis and stability of the asiRNAs. Such cell penetrating asiRNAs (cp-asiRNAs) are able to be delivered into the cell in the absence of a delivery vehicle.

Twenty potential cp-asiRNAs (Table 4) were screened for MyD88 mRNA inhibition in HeLa cells. Each potential cp-asiRNA was incubated with HeLa cells at 1 µM without a delivery vehicle and MyD88 mRNA levels were measured by Real-Time PCR.

**Table 4. Modified asiRNA sequences tested for self-delivery and MyD88 inhibition.**

| **m = 2'-O-Methyl RNA, * = phosphorothioate bond.** |
|---|
| cp-asiMyD88#26-1(S) : 5' GUGACUUCCAGACC*A*A*cholesterol 3' |
| cp-asiMyD88#26-1(AS) : 5' UUGGUCUGGAAGUCACA*U*U*C*C 3' |
| cp-asiMyD88#26-2(S) : 5' GUGACUUCCAGACC*A*A*cholesterol 3' |
| cp-asiMyD88#26-2(AS) : 5' UUGGUCUGGAAGUCACA*U*mU*mC*mC 3' |
| cp-asiMyD88#26-3(S) : 5' GUGACUUCCAGACC*A*A*cholesterol 3' |
| cp-asiMyD88#26-3(AS) : 5' UUGGUCUGGAAGUCACmA*mU*mU*mC*mC 3' |
| cp-asiMyD88#26-4(S) : 5' GUGACUUCCAGACC*A*A*cholesterol 3' |
| cp-asiMyD88#26-4(AS) : 5' UUGGUCUGGAAGUCmAmCmA*mU*U*C*C 3' |
| cp-asiMyD88#26-5(S) : 5' GUGACUUCCAGACC*A*A*cholesterol 3' |
| cp-asiMyD88#26-5(AS) : 5' UUGGUCUGGAAGUCmAmCA*U*U*C*C 3' |
| cp-asiMyD88#26-6(S) : 5' mGUmGAmCUmUCmCAmGAmCC*mA*A*cholesterol 3' |
| cp-asiMyD88#26-6(AS) : 5' UUGGUCUGGAAGUCACA*U*U*C*C 3' |
| cp-asiMyD88#26-7(S) : 5' mGUmGAmCUmUCmCAmGAmCC*mA*A*cholesterol 3' |
| cp-asiMyD88#26-7(AS) : 5' UUGGUCUGGAAGUCACA*U*mU*mC*mC 3' |
| cp-asiMyD88#26-8(S) : 5' mGUmGAmCUmUCmCAmGAmCC*mA*A*cholesterol 3' |
| cp-asiMyD88#26-8(AS) : 5' UUGGUCUGGAAGUCACmA*mU*mU*mC*mC 3' |
| cp-asiMyD88#26-9(S) : 5' mGUmGAmCUmUCmCAmGAmCC*mA*A*cholesterol 3' |
| cp-asiMyD88#26-9(AS) : 5' UUGGUCUGGAAGUCmAmCmA*mU*U*C*C 3' |
| cp-asiMyD88#26-10(S) : 5' mGUmGAmCUmUCmCAmGAmCC*mA*A*cholesterol 3' |
| cp-asiMyD88#26-10(AS) : 5' UUGGUCUGGAAGUCmAmCA*U*U*C*C 3' |
| cp-asiMyD88#27-1(S) : 5' UGUGACUUCCAGAC*C*A*cholesterol 3' |
| cp-asiMyD88#27-1(AS) : 5' UGGUCUGGAAGUCACAU*U*C*C*U 3' |
| cp-asiMyD88#27-2(S) : 5' UGUGACUUCCAGAC*C*A*cholesterol 3' |
| cp-asiMyD88#27-2(AS) : 5' UGGUCUGGAAGUCACAU*U*mC*mC*mU 3' |
| cp-asiMyD88#27-3(S) : 5' UGUGACUUCCAGAC*C*A*cholesterol 3' |
| cp-asiMyD88#27-3(AS) : 5' UGGUCUGGAAGUCACAmU*mU*mC*mC*mU 3' |
| cp-asiMyD88#27-4(S) : 5' UGUGACUUCCAGAC*C*A*cholesterol 3' |
| cp-asiMyD88#27-4(AS) : 5' UGGUCUGGAAGUCAmCmAmU*mU*C*C*U 3' |
| cp-asiMyD88#27-5(S) : 5' UGUGACUUCCAGAC*C*A*cholesterol 3' |
| cp-asiMyD88#27-5(AS) : 5' UGGUCUGGAAGUCAmCmAU*U*C*C*U 3' |
| cp-asiMyD88#27-6(S) : 5' mUGmUGmACmUUmCCmAGmAC*mC*A*cholesterol 3' |
| cp-asiMyD88#27-6(AS) : 5' UGGUCUGGAAGUCACAU*U*C*C*U 3' |
| cp-asiMyD88#27-7(S) : 5' mUGmUGmACmUUmCCmAGmAC*mC*A*cholesterol 3' |
| cp-asiMyD88#27-7(AS) : 5' UGGUCUGGAAGUCACAU*U*mC*mC*mU 3' |
| cp-asiMyD88#27-8(S) : 5' mUGmUGmACmUUmCCmAGmAC*mC*A*cholesterol 3' |
| cp-asiMyD88#27-8(AS) : 5' UGGUCUGGAAGUCACAmU*mU*mC*mC*mU 3' |
| cp-asiMyD88#27-9(S) : 5' mUGmUGmACmUUmCCmAGmAC*mC*A*cholesterol 3' |
| cp-asiMyD88#27-9(AS) : 5' UGGUCUGGAAGUCAmCmAmU*mU*C*C*U 3' |
| cp-asiMyD88#27-10(S) : 5' mUGmUGmACmUUmCCmAGmAC*mC*A*cholesterol 3' |
| cp-asiMyD88#27-10(AS) : 5' UGGUCUGGAAGUCAmCmAU*U*C*C*U 3' |

HeLa cells (ATCC) that had been cultured in Dulbecco's modified Eagle's medium (Gibco) containing 10% fetal bovine serum (Gibco) and 100 µg/ml penicillin/streptomycin in a 100 mm cell culture dish.

The potential cp-asiRNAs listed in Table 4 were incubated at 95 °C for 2 minutes and at 37 °C for 1 hour in OPTI-MEM buffer (Gibco). Proper strand annealing was confirmed via gel electrophoresis.

One day prior to transfection, 2.0 x 10⁴ HeLa cells were seeded in 24-well plates. Immediately before treatment, the HeLa cells were washed with 1x DPBS (Gibco) then cultured in the presence of the potential cp-asiRNAs in OPTI-MEM buffer for 24 hours, at which point the asiRNA-containing OPTI-MEM media was replaced with a serum-containing media.

After 48 hours of asiRNA treatment, the level of MyD88 mRNA expression was determined.

The level of MyD88 inhibition by each of the 20 potential cp-asiRNAs is provided Figure 6. Of the potential cp-asiRNAs tested, cp-asiRNA(26)-1 and cp-asiRNA(27)-7 exhibited the highest levels of MyD88 inhibition.

### Example 7: Inhibition of MyD88 protein using MyD88-specific cp-asiRNAs

The efficacy of cp-asiRNAs for the inhibition of MyD88 protein was tested. Each potential cp-asiRNA was incubated with HeLa cells at 1 µM without a delivery vehicle and MyD88 protein levels were measured by western blot.

HeLa cells (ATCC) were used that had been cultured in Dulbecco's modified Eagle's medium (Gibco) containing 10% fetal bovine serum (Gibco) and 100 µg/ml penicillin/streptomycin in a 100 mm cell culture dish. The cp-asiRNAs were incubated at 95 °C for 2 minutes and at 37 °C for 1 hour in OPTI-MEM buffer (Gibco). Proper strand annealing was confirmed via gel electrophoresis. One day prior to transfection, 5.0 x 10⁴ HeLa cells were seeded in 12-well plates. Immediately before treatment, the HeLa cells were washed with 1x DPBS (Gibco) then cultured in the presence of the potential cp-asiRNAs in OPTI-MEM buffer for 24 hours, at which point the asiRNA-containing OPTI-MEM media was replaced with a serum-containing media.

After 48 hours of asiRNA treatment, the level of MyD88 protein expression was determined via western blot. Briefly, the treated HeLa cells were lysed with RIPA buffer (GE). 30 µg of the total protein extract were loaded onto a 12% SDS-PAGE gel and electrophoresed at 120 V. After electrophoresis, the proteins were transferred to PVDF membrane (Bio-rad) already activated by methanol (Merck) for 1 hour at 300 mA. The membrane was blocked for 1 hour at the room temperature with 5% skim milk (Seoul Milk) and then incubated overnight at 4°C in 5% skim milk containing anti-MyD88 antibody (Abcam) and anti-γ-tubulin (Bethyl). The membrane was then washed with 1x TBST for 10 minutes three times and was incubated for 1 hour at the room temperature in 5% skim milk with HRP-conjugated secondary antibody. The membrane was washed with 1x TBST for 10 minutes and treated with 1x ECL for 1 minute. The MyD88 and γ-tubulin bands were then imaged using a Chemidoc instrument (Bio-rad).

The results of the western blot assay are depicted in Figure 7. All cp-asiMyD88(27)-4 and cp-asiMyD88(27)-7 incubated cell lines exhibited 60% or more of MyD88 protein inhibition (Figure 7).

### Example 8: Inhibition of MyD88 protein using MyD88-specific cp-asiRNAs

A variety of potential cp-asiMyD88 structures having different number of phosphorothioate bond in antisense strand were synthesized and tested for their ability to inhibit MyD88 expression (Table 5).

**Table 5. Additional cp-asiRNA sequences.**

| **m = 2'-O-Methyl RNA, * = phosphorothioate bond.** |
|---|
| cp-asiMyD88#26-11(S) : 5' GUGACUUCCAGACC*A*A*cholesterol 3' |
| cp-asiMyD88#26-11(AS) : 5' UUGGUCUGGAAGUCA*C*A*U*U*C*C 3' |
| cp-asiMyD88#26-12(S) : 5' GUGACUUCCAGACC*A*A*cholesterol 3' |
| cp-asiMyD88#26-12(AS) : 5' UUGGUCUGGAAGUCA*C*mA*mU*mU*mC*mC 3' |
| cp-asiMyD88#26-13(S) : 5' GUGACUUCCAGACC*A*A*cholesterol 3' |
| cp-asiMyD88#26-13(AS) : 5' UUGGUCUGGAAGUCmA*mC*mA*mU*U*C*C 3' |
| cp-asiMyD88#26-14(S) : 5' GUGACUUCCAGACC*A*A*cholesterol 3' |
| cp-asiMyD88#26-14(AS) : 5' UUGGUCUGGAAGUCmA*mC*A*U*U*C*C 3' |
| cp-asiMyD88#26-15(S) : 5' mGUmGAmCUmUCmCAmGAmCC*mA*A*cholesterol 3' |
| cp-asiMyD88#26-15(AS) : 5' UUGGUCUGGAAGUCA*C*A*U*U*C*C 3' |
| cp-asiMyD88#26-16(S) : 5' mGUmGAmCUmUCmCAmGAmCC*mA*A*cholesterol 3' |
| cp-asiMyD88#26-16(AS) : 5' UUGGUCUGGAAGUCA*C*mA*mU*mU*mC*mC 3' |
| cp-asiMyD88#26-17(S) : 5' mGUmGAmCUmUCmCAmGAmCC*mA*A*cholesterol 3' |
| cp-asiMyD88#26-17(AS) : 5' UUGGUCUGGAAGUCmA*mC*mA*mU*U*C*C 3' |
| cp-asiMyD88#26-18(S) : 5' mGUmGAmCUmUCmCAmGAmCC*mA*A*cholesterol 3' |
| cp-asiMyD88#26-18(AS) : 5' UUGGUCUGGAAGUCmA*mC*A*U*U*C*C 3' |
| cp-asiMyD88#27-11(S) : 5' UGUGACUUCCAGAC*C*A*cholesterol 3' |
| cp-asiMyD88#27-11(AS) : 5' UGGUCUGGAAGUCAC*A*U*U*C*C*U 3' |
| cp-asiMyD88#27-12(S) : 5' UGUGACUUCCAGAC*C*A*cholesterol 3' |
| cp-asiMyD88#27-12(AS) : 5' UGGUCUGGAAGUCAmC*mA*mU*mU*C*C*U 3' |
| cp-asiMyD88#27-13(S) : 5' mUGmUGmACmUUmCCmAGmAC*mC*A*cholesterol 3' |
| cp-asiMyD88#27-13(AS) : 5' UGGUCUGGAAGUCAC*A*U*U*mC*mC*mU 3' |
| cp-asiMyD88#27-14(S) : 5' mUGmUGmACmUUmCCmAGmAC*mC*A*cholesterol 3' |
| cp-asiMyD88#27-14(AS) : 5' UGGUCUGGAAGUCAmC*mA*mU*mU*C*C*U 3' |

The ability of 1 µM of each of the potential cp-asiRNAs listed in Table 5 to inhibit MyD88 mRNA in HeLa cells was tested. HeLa cells (ATCC) were used that had been cultured in Dulbecco's modified Eagle's medium (Gibco) containing 10% fetal bovine serum (Gibco) and 100 µg/ml penicillin / streptomycin in a 100 mm cell culture dish. The potential cp-asiRNAs listed in Table 5 were incubated at 95 °C for 2 minutes and at 37 °C for 1 hour in OPTI-MEM buffer (Gibco). Proper strand annealing was confirmed via gel electrophoresis. One day prior to transfection, 2.0 x 10⁴ HeLa cells were seeded in 24-well plates. Immediately before treatment, the HeLa cells were washed with 1x DPBS (Gibco) then cultured in the presence of the potential cp-asiRNAs in OPTI-MEM buffer for 24 hours, at which point the asiRNA-containing OPTI-MEM media was replaced with a serum-containing media. After 48 hours of asiRNA treatment, the level of MyD88 mRNA expression was determined.

As seen in Figure 8, MyD88 mRNA potential cp-asiRNA(26) containing 3 phosphorothioate bond on sense strand and 4 phosphorothioate bond on antisense strand, cp-asiRNA(27) containing 3 phosphorothioate bond on sense strand and three 2'-O-Methylation and 4 phosphorothioate bond on antisense strand exhibited the highest levels of MyD88 inhibition.

### Example 9: Inhibition of MyD88 protein using additional MyD88-specific cp-asiRNAs

The efficacy of cp-asiRNAs for the inhibition of MyD88 protein were tested. Each potential cp-asiRNA was incubated with HeLa cells at 3uM without a delivery vehicle and MyD88 protein levels were measured by western blot. HeLa cells (ATCC) were used that had been cultured in Dulbecco's modified Eagle's medium (Gibco) containing 10% fetal bovine serum (Gibco) and 100 µg/ml penicillin / streptomycin in a 100 mm cell culture dish. The cp-asiRNAs were incubated at 95 °C for 2 minutes and at 37 °C for 1 hour in OPTI-MEM buffer (Gibco). Proper strand annealing was confirmed via gel electrophoresis. One day prior to transfection, 5.0 x 10⁴ HeLa cells were seeded in 12-well plates. Immediately before treatment, the HeLa cells were washed with 1x DPBS (Gibco) then cultured in the presence of the potential cp-asiRNAs in OPTI-MEM buffer for 24 hours, at which point the asiRNA-containing OPTI-MEM media was replaced with a serum-containing media.

After 48 hours of asiRNA treatment, the level of MyD88 protein expression was determined via western blot. Briefly, the treated HeLa cells were lysed with RIPA buffer (GE). 30 µg of the total protein extract were loaded onto a 12% SDS-PAGE gel and electrophoresed at 120 V. After electrophoresis, the proteins were transferred to PVDF membrane (Bio-rad) already activated by methanol (Merck) for 1 hour at 300 mA. The membrane was blocked for 1 hour at the room temperature with 5% skim milk (Seoul Milk) and then incubated overnight at 4°C in 5% skim milk containing anti-MyD88 antibody (Abcam) and anti-γ-tubulin (Bethyl). The membrane was then washed with 1x TBST for 10 minutes three times and was incubated for 1 hour at the room temperature in 5% skim milk with HRP-conjugated secondary antibody. The membrane was washed with 1x TBST for 10 minutes and treated with 1x ECL for 1 minute. The MyD88 and γ-tubulin bands were then imaged using a Chemidoc instrument (Bio-rad).

The results of the western blot assay are depicted in Figure 9. All cp-asiMyD88 incubated cell lines exhibited 50% or more of MyD88 protein inhibition. In addition, the cp-asiMyD88(26)-10 and cp-asiMyD88(27)-2 were shown to have a higher efficiency in the MyD88 inhibition ability than other cp-asiMD88s (Figure 9).

### Example 10: additional MyD88 cp-asiRNA structures

A variety of potential cp-asiMyD88 structures having different strand lengths and numbers of 2'-O-methylation modifications and phosphorothioate bonds were synthesized and tested for their ability to inhibit MyD88 expression (Table 6).

**Table 6. Additional cp-asiRNA sequences(m = 2'-O-Methyl RNA, * = phosphorothioate bond).**

| |
|---|
| cp-asiMyD88#26-13(S) : 5' GUGACUUCCAGACC*A*A*cholesterol 3' |
| cp-asiMyD88#26-13(AS) : 5' UUGGUCUGGAAGUCmA*mC*mA*mU*U*C*C 3' |
| cp-asiMyD88#26-19(S) : 5' GUGACUUCCAGACC*A*A*cholesterol 3' |
| cp-asiMyD88#26-19(AS) : 5' UUGGUCUGGAAGU*C*mA*mC*mA*mU*U 3' |
| cp-asiMyD88#27-14(S) : 5' mUGmUGmACmUUmCCmAGmAC*mC*A*cholesterol 3' |
| cp-asiMyD88#27-14(AS) : 5' UGGUCUGGAAGUCAmC*mA*mU*mU*C*C*U 3' |
| cp-asiMyD88#27-15(S) : 5' mUGmUGmACmUUmCCmAGmAC*mC*A*cholesterol 3' |
| cp-asiMyD88#27-15(AS) : 5' UGGUCUGGAAGUC*A*mC*mA*mU*mU*C 3' |

The ability of 1 µM or 3 µM of each of the potential cp-asiRNAs listed in Table 6 to inhibit MyD88 mRNA in HeLa cells was tested. HeLa cells (ATCC) were used that had been cultured in Dulbecco's modified Eagle's medium (Gibco) containing 10% fetal bovine serum (Gibco) and 100 µg/ml penicillin/streptomycin in a 100 mm cell culture dish. The potential cp-asiRNAs listed in Table 6 were incubated at 95 °C for 2 minutes and at 37 °C for 1 hour in OPTI-MEM buffer (Gibco). Proper strand annealing was confirmed via gel electrophoresis. One day prior to transfection, 2.0 x 10⁴ HeLa cells were seeded in 24-well plates. Immediately before treatment, the HeLa cells were washed with 1x DPBS (Gibco) then cultured in the presence of the potential cp-asiRNAs in OPTI-MEM buffer for 24 hours, at which point the asiRNA-containing OPTI-MEM media was replaced with a serum-containing media. After 48 hours of asiRNA treatment, the level of MyD88 mRNA expression was determined.

As seen in Figure 10, MyD88 mRNA potential cp-asiRNA(26) containing four 2'-O-Methylation and six phosphorothioate bond on 21 nucleotide antisense strand, cp-asiRNA(27) containing four 2'-O-Methylation and six phosphorothioate bond on 19 nucleotide antisense strand exhibited the highest levels of MyD88 inhibition.

### Example 11: Inhibition of MyD88 protein using additional MyD88-specific cp-asiRNAs

The efficacy of cp-asiRNAs for the inhibition of MyD88 protein were tested. Each potential cp-asiRNA was incubated with HeLa cells at 1 µM and 3 µM without a delivery vehicle and MyD88 protein levels were measured by western blot. HeLa cells (ATCC) were used that had been cultured in Dulbecco's modified Eagle's medium (Gibco) containing 10% fetal bovine serum (Gibco) and 100 µg/ml penicillin/streptomycin in a 100 mm cell culture dish. The cp-asiRNAs were incubated at 95 °C for 2 minutes and at 37 °C for 1 hour in OPTI-MEM buffer (Gibco). Proper strand annealing was confirmed via gel electrophoresis.

One day prior to transfection, 5.0 x 10⁴ HeLa cells were seeded in 12-well plates. Immediately before treatment, the HeLa cells were washed with 1x DPBS (Gibco) then cultured in the presence of the potential cp-asiRNAs in OPTI-MEM buffer for 24 hours, at which point the asiRNA-containing OPTI-MEM media was replaced with a serum-containing media. After 48 hours of asiRNA treatment, the level of MyD88 protein expression was determined via western blot. Briefly, the treated HeLa cells were lysed with RIPA buffer (GE). 30 µg of the total protein extract were loaded onto a 12% SDS-PAGE gel and electrophoresed at 120 V. After electrophoresis, the proteins were transferred to PVDF membrane (Bio-rad) already activated by methanol (Merck) for 1 hour at 300 mA. The membrane was blocked for 1 hour at the room temperature with 5% skim milk (Seoul Milk) and then incubated overnight at 4°C in 5% skim milk containing anti-MyD88 antibody (Abcam) and anti-γ-tubulin (Bethyl). The membrane was then washed with 1x TBST for 10 minutes three times and was incubated for 1 hour at the room temperature in 5% skim milk with HRP-conjugated secondary antibody. The membrane was washed with 1x TBST for 10 minutes and treated with 1x ECL for 1 minute. The MyD88 and γ-tubulin bands were then imaged using a Chemidoc instrument (Bio-rad).

The results of the western blot assay are depicted in Figure 11. All 3 µM cp-asiMyD88 incubated cell lines exhibited 50% or more of MyD88 protein inhibition. In addition, the cp-asiMyD88(26)-13 were shown to have a higher efficiency in the MyD88 inhibition ability than other cp-asiMD88s (Figure 11).

### Example 12: Screening for Toll-like receptor 3 specific asymmetric small interfering RNAs

To identify asymmetric small interfering RNAs (asiRNAs) that inhibit Toll-like receptor 3 with high efficiency, 100 asiRNAs were synthesized and screened. The nucleic acid sequences of the screened asiRNAs are provided in Table 7.

**Table 7: Nucleic acid sequences for exemplary Toll-like receptor 3 targeting asiRNA.**

| **TLR3 asiRNA Sequence** | | | |
|---|---|---|---|
| **Name** | **Sequence (5'-3')** | **Name** | **Sequence (5'-3')** |
| TLR3asiRNA S(1) | AUCUUUCCUACAACAA | TLR3asiRNA AS(1) | UUGUUGUAGGAAAGAUCGAGC |
| TLR3asiRNA S(2) | UCUUUCCUACAACAAC | TLR3asiRNA AS(2) | GUUGUUGUAGGAAAGAUCGAG |
| TLR3asiRNA S(3) | GGCCCUUAAAAAUGUG | TLR3asiRNA AS(3) | CACAUUUUUAAGGGCCACCCU |
| TLR3asiRNA S(4) | GCCCUUAAAAAUGUGG | TLR3asiRNA AS(4) | CCACAUUUUUAAGGGCCACCC |
| TLR3asiRNA S(5) | CCCUUAAAAAUGUGGA | TLR3asiRNA AS(5) | UCCACAUUUUUAAGGGCCACC |
| TLR3asiRNA S(6) | CCUUAAAAAUGUGGAU | TLR3asiRNA AS(6) | AUCCACAUUUUUAAGGGCCAC |
| TLR3asiRNA S(7) | CUUAAAAAUGUGGAUA | TLR3asiRNA AS(7) | UAUCCACAUUUUUAAGGGCCA |
| TLR3asiRNA S(8) | UCGUAACUUGACCAUU | TLR3asiRNA AS(8) | AAUGGUCAAGUUACGAAGAGG |
| TLR3asiRNA S(9) | CGUAACUUGACCAUUC | TLR3asiRNA AS(9) | GAAUGGUCAAGUUACGAAGAG |
| TLR3asiRNA S(10) | GUAACUUGACCAUUCU | TLR3asiRNA AS(10) | AGAAUGGUCAAGUUACGAAGA |
| TLR3asiRNA S(11) | UAACUUGACCAUUCUG | TLR3asiRNA AS(11) | CAGAAUGGUCAAGUUACGAAG |
| TLR3asiRNA S(12) | AACUUGACCAUUCUGG | TLR3asiRNA AS(12) | CCAGAAUGGUCAAGUUACGAA |
| TLR3asiRNA S(13) | ACUUGACCAUUCUGGA | TLR3asiRNA AS(13) | UCCAGAAUGGUCAAGUUACGA |
| TLR3asiRNA S(14) | AACAACAACAUAGCCA | TLR3asiRNA AS(14) | UGGCUAUGUUGUUGUUGCUUA |
| TLR3asiRNA S(15) | ACAACAACAUAGCCAA | TLR3asiRNA AS(15) | UUGGCUAUGUUGUUGUUGCUU |
| TLR3asiRNA S(16) | CAACAACAUAGCCAAC | TLR3asiRNA AS(16) | GUUGGCUAUGUUGUUGUUGCU |
| TLR3asiRNA S(17) | AACAACAUAGCCAACA | TLR3asiRNA AS(17) | UGUUGGCUAUGUUGUUGUUGC |
| TLR3asiRNA S(18) | ACAACAUAGCCAACAU | TLR3asiRNA AS(18) | AUGUUGGCUAUGUUGUUGUUG |
| TLR3asiRNA S(19) | CAACAUAGCCAACAUA | TLR3asiRNA AS(19) | UAUGUUGGCUAUGUUGUUGUU |
| TLR3asiRNA S(20) | AACAUAGCCAACAUAA | TLR3asiRNA AS(20) | UUAUGUUGGCUAUGUUGUUGU |
| TLR3asiRNA S(21) | ACAUAGCCAACAUAAA | TLR3asiRNA AS(21) | UUUAUGUUGGCUAUGUUGUUG |
| TLR3asiRNA S(22) | AUAGCCAACAUAAAUG | TLR3asiRNA AS(22) | CAUUUAUGUUGGCUAUGUUGU |
| TLR3asiRNA S(23) | UAGCCAACAUAAAUGA | TLR3asiRNA AS(23) | UCAUUUAUGUUGGCUAUGUUG |
| TLR3asiRNA S(24) | AAUCUCUCAAAUUUUG | TLR3asiRNA AS(24) | CAAAAUUUGAGAGAUUGGUCU |
| TLR3asiRNA S(25) | UGCACUCUGUUUGCGA | TLR3asiRNA AS(25) | UCGCAAACAGAGUGCAUGGUU |
| TLR3asiRNA S(26) | GCACUCUGUUUGCGAA | TLR3asiRNA AS(26) | UUCGCAAACAGAGUGCAUGGU |
| TLR3asiRNA S(27) | CACUCUGUUUGCGAAG | TLR3asiRNA AS(27) | CUUCGCAAACAGAGUGCAUGG |
| TLR3asiRNA S(28) | ACUCUGUUUGCGAAGA | TLR3asiRNA AS(28) | UCUUCGCAAACAGAGUGCAUG |
| TLR3asiRNA S(29) | CUCUGUUUGCGAAGAG | TLR3asiRNA AS(29) | CUCUUCGCAAACAGAGUGCAU |
| TLR3asiRNA S(30) | UCUGUUUGCGAAGAGG | TLR3asiRNA AS(30) | CCUCUUCGCAAACAGAGUGCA |
| TLR3asiRNA S(31) | CUGUUUGCGAAGAGGA | TLR3asiRNA AS(31) | UCCUCUUCGCAAACAGAGUGC |
| TLR3asiRNA S(32) | UGUUUGCGAAGAGGAA | TLR3asiRNA AS(32) | UUCCUCUUCGCAAACAGAGUG |
| TLR3asiRNA S(33) | GUUUGCGAAGAGGAAU | TLR3asiRNA AS(33) | AUUCCUCUUCGCAAACAGAGU |
| TLR3asiRNA S(34) | UUUGCGAAGAGGAAUG | TLR3asiRNA AS(34) | CAUUCCUCUUCGCAAACAGAG |
| TLR3asiRNA S(35) | UUGCGAAGAGGAAUGU | TLR3asiRNA AS(35) | ACAUUCCUCUUCGCAAACAGA |
| TLR3asiRNA S(36) | UGCGAAGAGGAAUGUU | TLR3asiRNA AS(36) | AACAUUCCUCUUCGCAAACAG |
| TLR3asiRNA S(37) | GCGAAGAGGAAUGUUU | TLR3asiRNA AS(37) | AAACAUUCCUCUUCGCAAACA |
| TLR3asiRNA S(38) | CGAAGAGGAAUGUUUA | TLR3asiRNA AS(38) | UAAACAUUCCUCUUCGCAAAC |
| TLR3asiRNA S(39) | GAAGAGGAAUGUUUAA | TLR3asiRNA AS(39) | UUAAACAUUCCUCUUCGCAAA |
| TLR3asiRNA S(40) | AAGAGGAAUGUUUAAA | TLR3asiRNA AS(40) | UUUAAACAUUCCUCUUCGCAA |
| TLR3asiRNA S(41) | AGAGGAAUGUUUAAAU | TLR3asiRNA AS(41) | AUUUAAACAUUCCUCUUCGCA |
| TLR3asiRNA S(42) | GAGGAAUGUUUAAAUC | TLR3asiRNA AS(42) | GAUUUAAACAUUCCUCUUCGC |
| TLR3asiRNA S(43) | AGGAAUGUUUAAAUCU | TLR3asiRNA AS(43) | AGAUUUAAACAUUCCUCUUCG |
| TLR3asiRNA S(44) | GGAAUGUUUAAAUCUC | TLR3asiRNA AS(44) | GAGAUUUAAACAUUCCUCUUC |
| TLR3asiRNA S(45) | CUUGAACUGGCCAGUU | TLR3asiRNA AS(45) | AACUGGCCAGUUCAAGAUGCA |
| TLR3asiRNA S(46) | UUGAACUGGCCAGUUC | TLR3asiRNA AS(46) | GAACUGGCCAGUUCAAGAUGC |
| TLR3asiRNA S(47) | UGAACUGGCCAGUUCA | TLR3asiRNA AS(47) | UGAACUGGCCAGUUCAAGAUG |
| TLR3asiRNA S(48) | GAACUGGCCAGUUCAG | TLR3asiRNA AS(48) | CUGAACUGGCCAGUUCAAGAU |
| TLR3asiRNA S(49) | AACUGGCCAGUUCAGA | TLR3asiRNA AS(49) | UCUGAACUGGCCAGUUCAAGA |
| TLR3asiRNA S(50) | ACUGGCCAGUUCAGAA | TLR3asiRNA AS(50) | UUCUGAACUGGCCAGUUCAAG |
| TLR3asiRNA S(51) | CUGGCCAGUUCAGAAA | TLR3asiRNA AS(51) | UUUCUGAACUGGCCAGUUCAA |
| TLR3asiRNA S(52) | UGGCCAGUUCAGAAAG | TLR3asiRNA AS(52) | CUUUCUGAACUGGCCAGUUCA |
| TLR3asiRNA S(53) | GGCCAGUUCAGAAAGA | TLR3asiRNA AS(53) | UCUUUCUGAACUGGCCAGUUC |
| TLR3asiRNA S(54) | GCCAGUUCAGAAAGAA | TLR3asiRNA AS(54) | UUCUUUCUGAACUGGCCAGUU |
| TLR3asiRNA S(55) | CCAGUUCAGAAAGAAC | TLR3asiRNA AS(55) | GUUCUUUCUGAACUGGCCAGU |
| TLR3asiRNA S(56) | CAGUUCAGAAAGAACG | TLR3asiRNA AS(56) | CGUUCUUUCUGAACUGGCCAG |
| TLR3asiRNA S(57) | AGUUCAGAAAGAACGG | TLR3asiRNA AS(57) | CCGUUCUUUCUGAACUGGCCA |
| TLR3asiRNA S(58) | GUUCAGAAAGAACGGA | TLR3asiRNA AS(58) | UCCGUUCUUUCUGAACUGGCC |
| TLR3asiRNA S(59) | UUCAGAAAGAACGGAU | TLR3asiRNA AS(59) | AUCCGUUCUUUCUGAACUGGC |
| TLR3asiRNA S(60) | UCAGAAAGAACGGAUA | TLR3asiRNA AS(60) | UAUCCGUUCUUUCUGAACUGG |
| TLR3asiRNA S(61) | AAUUGCAAGUAGCACU | TLR3asiRNA AS(61) | AGUGCUACUUGCAAUUUAUGA |
| TLR3asiRNA S(62) | AUUGCAAGUAGCACUU | TLR3asiRNA AS(62) | AAGUGCUACUUGCAAUUUAUG |
| TLR3asiRNA S(63) | UUGCAAGUAGCACUUG | TLR3asiRNA AS(63) | CAAGUGCUACUUGCAAUUUAU |
| TLR3asiRNA S(64) | UGCAAGUAGCACUUGG | TLR3asiRNA AS(64) | CCAAGUGCUACUUGCAAUUUA |
| TLR3asiRNA S(65) | GCAAGUAGCACUUGGA | TLR3asiRNA AS(65) | UCCAAGUGCUACUUGCAAUUU |
| TLR3asiRNA S(66) | CAAGUAGCACUUGGAU | TLR3asiRNA AS(66) | AUCCAAGUGCUACUUGCAAUU |
| TLR3asiRNA S(67) | AAGUAGCACUUGGAUC | TLR3asiRNA AS(67) | GAUCCAAGUGCUACUUGCAAU |
| TLR3asiRNA S(68) | UGCCCCCUUUGAACUC | TLR3asiRNA AS(68) | GAGUUCAAAGGGGGCACUGUC |
| TLR3asiRNA S(69) | UCUGGGAACAUUUCUC | TLR3asiRNA AS(69) | GAGAAAUGUUCCCAGACCCAA |
| TLR3asiRNA S(70) | CAGCAUCAAAAGAAGC | TLR3asiRNA AS(70) | GCUUCUUUUGAUGCUGUUAAC |
| TLR3asiRNA S(71) | CACGUGUGAAAGUAUU | TLR3asiRNA AS(71) | AAUACUUUCACACGUGCAAUC |
| TLR3asiRNA S(72) | GUCUCACCUCCACAUC | TLR3asiRNA AS(72) | GAUGUGGAGGUGAGACAGACC |
| TLR3asiRNA S(73) | UGUCUCACCUCCACAU | TLR3asiRNA AS(73) | AUGUGGAGGUGAGACAGACCC |
| TLR3asiRNA S(74) | AGAUUCAAGGUACAUC | TLR3asiRNA AS(74) | GAUGUACCUUGAAUCUUUUGC |
| TLR3asiRNA S(75) | GGAAACACGCAAACCC | TLR3asiRNA AS(75) | GGGUUUGCGUGUUUCCAGAGC |
| TLR3asiRNA S(76) | UGGAAACACGCAAACC | TLR3asiRNA AS(76) | GGUUUGCGUGUUUCCAGAGCC |
| TLR3asiRNA S(77) | UUGAGAAACUAGAAAU | TLR3asiRNA AS(77) | AUUUCUAGUUUCUCAAGACCC |
| TLR3asiRNA S(78) | CUUGAGAAACUAGAAA | TLR3asiRNA AS(78) | UUUCUAGUUUCUCAAGACCCU |
| TLR3asiRNA S(79) | AACAUCCGUUGAGAAG | TLR3asiRNA AS(79) | CUUCUCAACGGAUGUUAUGAG |
| TLR3asiRNA S(80) | GUGCCCCCUUUGAACU | TLR3asiRNA AS(80) | AGUUCAAAGGGGGCACUGUCU |
| TLR3asiRNA S(81) | AGUGCCCCCUUUGAAC | TLR3asiRNA AS(81) | GUUCAAAGGGGGCACUGUCUU |
| TLR3asiRNA S(82) | CAGUGCCCCCCUUUGAA | TLR3asiRNA AS(82) | UUCAAAGGGGGCACUGUCUUU |
| TLR3asiRNA S(83) | GGAGGAUAUCUUUUUA | TLR3asiRNA AS(83) | UAAAAAGAUAUCCUCCAGCCC |
| TLR3asiRNA S(84) | UGGAGGAUAUCUUUUU | TLR3asiRNA AS(84) | AAAAAGAUAUCCUCCAGCCCU |
| TLR3asiRNA S(85) | ACUGAACCAUGCACUC | TLR3asiRNA AS(85) | GAGUGCAUGGUUCAGUUUAUA |
| TLR3asiRNA S(86) | UGAACCAUGCACUCUG | TLR3asiRNA AS(86) | CAGAGUGCAUGGUUCAGUUUA |
| TLR3asiRNA S(87) | GAACCAUGCACUCUGU | TLR3asiRNA AS(87) | ACAGAGUGCAUGGUUCAGUUU |
| TLR3asiRNA S(88) | AACCAUGCACUCUGUU | TLR3asiRNA AS(88) | AACAGAGUGCAUGGUUCAGUU |
| TLR3asiRNA S(89) | ACCAUGCACUCUGUUU | TLR3asiRNA AS(89) | AAACAGAGUGCAUGGUUCAGU |
| TLR3asiRNA S(90) | CCAUGCACUCUGUUUG | TLR3asiRNA AS(90) | CAAACAGAGUGCAUGGUUCAG |
| TLR3asiRNA S(91) | CAUGCACUCUGUUUGC | TLR3asiRNA AS(91) | GCAAACAGAGUGCAUGGUUCA |
| TLR3asiRNA S(92) | CUGCAUCUUGAACUGG | TLR3asiRNA AS(92) | CCAGUUCAAGAUGCAGUGAGA |
| TLR3asiRNA S(93) | ACUGCAUCUUGAACUG | TLR3asiRNA AS(93) | CAGUUCAAGAUGCAGUGAGAU |
| TLR3asiRNA S(94) | CACUGCAUCUUGAACU | TLR3asiRNA AS(94) | AGUUCAAGAUGCAGUGAGAUU |
| TLR3asiRNA S(95) | UCACUGCAUCUUGAAC | TLR3asiRNA AS(95) | GUUCAAGAUGCAGUGAGAUUU |
| TLR3asiRNA S(96) | UAAAUUGCAAGUAGCA | TLR3asiRNA AS(96) | UGCUACUUGCAAUUUAUGACG |
| TLR3asiRNA S(97) | AUAAAUUGCAAGUAGC | TLR3asiRNA AS(97) | GCUACUUGCAAUUUAUGACGA |
| TLR3asiRNA S(98) | CGUCAUAAAUUGCAAG | TLR3asiRNA AS(98) | CUUGCAAUUUAUGACGAAAGG |
| TLR3asiRNA S(99) | UCGUCAUAAAUUGCAA | TLR3asiRNA AS(99) | UUGCAAUUUAUGACGAAAGGC |
| TLR3asiRNA S(100) | UUCGUCAUAAAUUGCA | TLR3asiRNA AS(100) | UGCAAUUUAUGACGAAAGGCA |

The asiRNAs listed in Table 7 were incubated at 95 °C for 2 minutes and at 37 °C for 1 hour in annealing buffer (Bioneer Inc. Korea). Proper strand annealing was confirmed via gel electrophoresis using UV transilluminator. For the screen, 5X10³ HaCaT cells (ATCC) that had been cultured in Dulbecco's modified Eagle's medium (DMEM, Gibco) containing 10% fetal bovine serum (FBS, Gibco) and 100 units/ml Penicillin and 100 µg/ml Streptomycin in a 100 mm cell culture dish were seeded in 96well plates. The HaCaT cells were transfected with 0.1 nM of the asiRNAs using RNAiMAX (Invitrogen Inc.) according to the manufacturer's instructions. The TLR3 mRNA levels in the transfected cells were measured 24 hours after transfection using qRT-PCR. Specifically, total RNA were extracted using TOYOBO lysis reagent and then 1/5 volume of the reaction mixture was used for cDNA synthesis using the TOYOBO RT reagent (TOYOBO SuperPrep). The synthesized cDNA was diluted and then quantitative RT-PCR was performed using THUNDERBIRD^{®} Probe qPCR Mix (TOYOBO). Amplification of the target gene was detected using TLR3 TaqMan^{®} Probe (Hs01551078_m1) and 18S TaqMan^{®} Probe (Hs03928985_g1).

The expression level of TLR3 inhibition by each of the 100 asiRNAs is provided in Figure 12. 17 of the asiRNA sequences, asiRNA (13), asiRNA (25), asiRNA (26), asiRNA (28), asiRNA (32), asiRNA (33), asiRNA (37), asiRNA (38), asiRNA (39), asiRNA (53), asiRNA (58), asiRNA (60), asiRNA (71), asiRNA (77), asiRNA (78), asiRNA (82) and asiRNA (83), were selected for use in follow-up studies.

### Example 13: Chemical modification of asiRNAs for self-delivery

Chemical modifications were applied to the asiRNAs selected in Example 12 and the cellular delivery of the modified asiRNAs was tested in the absence of other delivery vehicle. As described below, certain of the modifications improved endocytosis and stability of asiRNAs. Such cell penetrating asiRNAs (cp-asiRNAs) are able to be delivered into the cell in the absence of a delivery vehicle. The expression of TLR3 mRNA by the cells is provided in Figures 13 and 14 and the TLR3 protein levels are provided in Figure 15, as determined using methods described above. The morphology of the cells is depicted in Figure 16.

Potential cp-asiRNA (Table 8) were screened for Toll-like receptor 3 (TLR3) mRNA inhibition in HaCaT cells. Each potential cp-asiRNA was incubated with HaCaT cells, human skin keratinocyte cell line, at 1 µM and 3 µM without a delivery vehicle and TLR3 expression levels were measured by qRT-PCR and western blot study.

**Table 8: Modified asiRNA sequences tested for self-delivery and TLR3 inhibition. (m= 2'-O-Methyl RNA. *= phosphorothioate bond).**

| Name | Sense(5' to 3') |
|---|---|
| TLR3cp-asiRNA S 25 | mUGmCAmCUmCUmGUmUUmGC*mG*A*cholesterol |
| TLR3cp-asiRNA AS 25(2,4) | UCGCAAACAGAGUGmCmAU*G*G*U*U |
| TLR3cp-asiRNA AS 25(4,4) | UCGCAAACAGAGUGmCmAmU*mG*G*U*U |
| TLR3cp-asiRNA AS 25(7,4) | UCGCAAACAGAGUGmCmAmU*mG*mG*mU*mU |
| TLR3cp-asiRNA S 28 | mACmUCmUGmUUmUGmCGmAA*mG*A*cholesterol |
| TLR3cp-asiRNA AS 28(2,4) | UCUUCGCAAACAGAmGmUG*C*A*U*G |
| TLR3cp-asiRNA AS 28(4,4) | UCUUCGCAAACAGAmGmUmG*mC*A*U*G |
| TLR3cp-asiRNA AS 28(7,4) | UCUUCGCAAACAGAmGmUmG*mC*mA*mU*mG |
| TLR3cp-asiRNA S 32 | mUGmUUmUGmCGmAAmGAmGG*mA*A*cholesterol |
| TLR3cp-asiRNA AS 32(2,4) | UUCCUCUUCGCAAAmCmAG*A*G*U*G |
| TLR3cp-asiRNA AS 32(4,4) | UUCCUCUUCGCAAAmCmAmG*mA*G*U*G |
| TLR3cp-asiRNA AS 32(7,4) | UUCCUCUUCGCAAAmCmAmG*mA*mG*mU*mG |
| TLR3cp-asiRNA S 33 | mGUmUUmGCmGAmAGmAGmGA*mA*U*cholesterol |
| TLR3cp-asiRNA AS 33(2,4) | AUUCCUCUUCGCAAmAmCA*G*A*G*U |
| TLR3cp-asiRNA AS 33(4,4) | AUUCCUCUUCGCAAmAmCmA*mG*A*G*U |
| TLR3cp-asiRNA AS 33(7,4) | AUUCCUCUUCGCAAmAmCmA*mG*mA*mG*mU |
| TLR3cp-asiRNA S 39 | mGAmAGmAGmGAmAUmGUmUU*mA*A*cholesterol |
| TLR3cp-asiRNA AS 39(2,4) | UUAAACAUUCCUCUmUmCG*C*A*A*A |
| TLR3cp-asiRNA AS 39(4,4) | UUAAACAUUCCUCUmUmCmG*mC*A*A*A |
| TLR3cp-asiRNA AS 39(7,4) | UUAAACAUUCCUCUmUmCmG*mC*mA*mA*mA |
| TLR3cp-asiRNA S 53 | mGGmCCmAGmUUmCAmGAmAA*mG*A*cholesterol |
| TLR3cp-asiRNA AS 53(2,4) | UCUUUCUGAACUGGmCmCA*G*U*U*C |
| TLR3cp-asiRNA AS 53(4,4) | UCUUUCUGAACUGGmCmCmA*mG*U*U*C |
| TLR3cp-asiRNA AS 53(7,4) | UCUUUCUGAACUGGmCmCmA*mG*mU*mU*mC |
| TLR3cp-asiRNA S 58 | mGUmUCmAGmAAmAGmAAmCG*mG*A*cholesterol |
| TLR3cp-asiRNA AS 58(2,4) | UCCGUUCUUUCUGAmAmCU*G*G*C*C |
| TLR3cp-asiRNA AS 58(4,4) | UCCGUUCUUUCUGAmAmCmU*mG*G*C*C |
| TLR3cp-asiRNA AS 58(7,4) | UCCGUUCUUUCUGAmAmCmU*mG*mG*mC*mC |
| TLR3cp-asiRNA S 60 | mUCmAGmAAmAGmAAmCGmGA*mU*A*cholesterol |
| TLR3cp-asiRNA AS 60(2,4) | UAUCCGUUCUUUCUmGmAA*C*U*G*G |
| TLR3cp-asiRNA AS 60(4,4) | UAUCCGUUCUUUCUmGmAmA*mC*U*G*G |
| TLR3cp-asiRNA AS 60(7,4) | UAUCCGUUCUUUCUmGmAmA*mC*mU*mG*mG |

HaCaT cells (ATCC) were cultured in Dulbecco's modified Eagle's medium (DMEM, Gibco) containing 10% fetal bovine serum (FBS, Gibco) and 100 units/ml Penicillin 100 µg/ml Streptomycin in a 100 mm cell culture dish. The potential cp-asiRNAs listed in Table 8 were incubated at 95 °C for 2 minutes and at 37 °C for 1 hour in Opti-MEM (Gibco). Proper strand annealing of the potential cp-asiRNAs was confirmed by gel electrophoresis.

On that day cp-asiRNAs treatment, 5 X10⁴ cells were seeded into 24 well plates and then cultured in the presence of the potential cp-asiRNAs in Opti-MEM for 24 hours, at which point the cp-asiRNA-containing Opti-MEM media was replaced with a serum-containing media. Twenty-four hours later, TLR3 mRNA levels in HaCaT cells were determined using qRT-PCR. Specifically, total RNA were extracted using RNAiPlus^{®} (TaKaRa) and then 500 ng of the reaction mixture was used for cDNA synthesis using the High-capacity cDNA reverse transcription kit (Applied Biosystems). The synthesized cDNA was diluted and then quantitative RT-PCR was performed using power SYBR green PCR master Mix (Applied Biosystems). The following primer sequences were used:

**Table 9: Primer sequences.**

| **Name** | | **Sequence (5'-3')** | **size** |
|---|---|---|---|
| Human GAPDH | Forward Reverse | GAG TCA ACG GAT TTG GTC GT | 186 |
| | | GAC AAG CTT CCC GTT CTC AG | |
| Human TLR3 (Toll-like receptor 3) | Forward Reverse | TGC CCC CTT TGA ACT CTT TT | 298 |
| | | AAA AAC ACC CGC CTC AAA GT | |

After 48 hours of cp-asiRNAs incubation, the level of TLR3 protein expression was determined via western blot. Briefly, the treated HaCaT cells were lysed with Mammalian protein Extraction Buffer (GE Healthcare). 10 µg of the total protein extract were loaded onto an 8% SDS-PAGE gel and electrophoresed at 120 V. After electrophoresis, the proteins were transferred to PVDF membrane (Bio-rad) already activated by methanol (Merck) for 1 hour at 300 mA. The membrane was blocked for 1 hour at the room temperature with 5% skim milk (Seoul Milk) and then incubated overnight at 4 °C in 5% skim milk containing anti-TLR3 antibody (Abcam) and anti-γ-tubulin antibody (Bethyl). The membrane was then washed with TBST for 10 minutes three times and was incubated for 1 hour at the room temperature in 5% skim milk with HRP-conjugated secondary antibody (Santa Cruz). The membrane was washed with TBST for 10 minutes and treated with ECL substrate (Thermo scientific). The Target protein bands were then imaged using a Chemidoc instrument (Bio-rad).

The levels of TLR3 inhibition by each of the 24 potential cp-asiRNAs is provided in Figures 17 and 18 From among the potential cp-asiRNAs tested, cp-asiTLR3 39 (2, 4) was selected for further study.

### Example 14: Additional TLR3 cp-asiRNA structures

Other potential cp-asiTLR3 structure having different strand length was synthesized and tested for its ability to inhibit TLR3 expression (Table 10)

**Table 10: Additional cp-asiRNA sequences (m= 2'-O-Methyl RNA. *= phosphorothioate bond).**

| Name | Sequence(5' to3') |
|---|---|
| TLR3cp-asiRNA S 39 | mGAmAGmAGmGAmAUmGUmUU*mA*A*cholesterol |
| TLR3cp-asiRNA AS 39 (19) | UUAAACAUUCCUCUmU*mC*G*C*A |
| TLR3cp-asiRNA AS 39(2,4) | UUAAACAUUCCUCUmUmCG*C*A*A*A |

The ability of dose dependent of each of the potential cp-asiRNAs listed in Table 10 to inhibit TLR3 expression in HaCaT cells was tested. HaCaT cells were cultured in Dulbecco's modified Eagle's medium (DMEM, Gibco) containing 10% fetal bovine serum (FBS, Gibco) and 100 units/ml Penicillin 100 µg/ml Streptomycin. The potential cp-asiRNAs listed in Table 10 were incubated at 95 °C for 2 minutes and at 37 °C for 1 hour in Opti-MEM (Gibco). Proper strand annealing of the potential cp-asiRNAs was confirmed by gel electrophoresis. On that day cp-asiRNAs treatment, 5 X10⁴ cells were seeded 24 well plates then cultured in the presence of the potential cp-asiRNAs in Opti-MEM for 24 hours, at which point the cp-asiRNA-containing Opti-MEM media was replaced with a serum-containing media. Twenty-four hours later, TLR3 expression levels in HaCaT cells were determined.

As seen the Figure 19, TLR3 expression potential cp-asiRNAs consist of 21 nucleotide antisense strands and potential cp-asiRNAs consist of 19 nucleotide antisense strands exhibited the similar levels of TLR3 inhibition. The cp-asiTLR3 (39) 21 and cp-asiTLR3 (39) 19 were selected for future experimentation.

The efficacy of cp-asiTLR3 (39) 21 and cp-asiTLR3 (39) 19 in low concentration on the production TLR3 protein was tested. The cp-asiRNAs were incubated at 95 °C for 2 minutes and at 37 °C for 1 hour in Opti-MEM (Gibco). Proper strand annealing of the potential cp-asiRNAs was confirmed by gel electrophoresis. HaCaT cells were cultured in Dulbecco's modified Eagle's medium (DMEM, Gibco) containing 10% fetal bovine serum (FBS, Gibco) and 100 units/ml Penicillin and 100ug/ml Streptomycin. On the day of treatment, 5X10⁴ HaCaT cells were seeded in 12-well plates then cultured in the presence of the potential cp-asiRNAs in Opti-MEM.

Twenty-four hours later, TLR3 protein levels in HaCaT were determined via western blot. Briefly, the treated HaCaT cells were lysed with Mammalian protein Extraction Buffer (GE Healthcare). 10ug of the total protein extract were loaded onto an 8% SDS-PAGE gel and electrophoresed at 120 V. After electrophoresis, the proteins were transferred to PVDF membrane (Bio-rad) already activated by methanol (Merck) for 1 hour at 300 mA. The membrane was blocked for 1 hour at the room temperature with 5% skim milk (Seoul Milk) and then incubated overnight at 4 °C in 5% skim milk containing anti-TLR3 antibody (Abcam) and anti-γ-tubulin antibody (Bethyl). The membrane was then washed with TBST for 10 minutes three times and was incubated for 1 hour at the room temperature in 5% skim milk with HRP-conjugated secondary antibody (Santa Cruz). The membrane was washed with TBST for 10 minutes and treated with ECL substrate (Thermo scientific). The Target protein bands were then imaged using a Chemidoc instrument (Bio-rad).

As seen in Figure 20, TLR3 expression potential cp-asiRNAs having 21 nucleotide antisense strands and potential cp-asiRNAs having 19 nucleotide antisense strands exhibited the similar levels of TLR3 inhibition in low concentration.

### Incorporation by Reference

All publications, patents, and patent applications mentioned herein are hereby incorporated by reference in their entirety as if each individual publication, patent or patent application was specifically and individually indicated to be incorporated by reference. In case of conflict, the present application, including any definitions herein, will control.

### Equivalents

Those skilled in the art will recognize, or be able to ascertain using no more than routine experimentation, many equivalents to the specific embodiments of the invention described herein. Such equivalents are intended to be encompassed by the following claims. Described, disclosed and exemplified herein are the following statements of invention:
1. An RNA complex comprising an antisense strand of at least 19 nucleotides (nt) in length having sequence complementarity to a MyD88 mRNA sequence and a sense strand of 15 to 17 nt in length having sequence complementarity to the antisense strand, wherein the antisense strand and the sense strand form a complex in which the 5' end of the antisense strand and the 3' end of the sense strand form a blunt end.
2. An RNA complex comprising an antisense strand of at least 19 nucleotides (nt) in length having sequence complementarity to a TLR3 mRNA sequence and a sense strand of 15 to 17 nt in length having sequence complementarity to the antisense strand, wherein the antisense strand and the sense strand form a complex in which the 5' end of the antisense strand and the 3' end of the sense strand form a blunt end.
3. The RNA complex of sentence 1 or 2, wherein the antisense strand is 19 to 21 nt in length.
4. The RNA complex of sentence 1 or 2, wherein the antisense strand is 19 nt in length.
5. The RNA complex of sentence 1 or 2, wherein the antisense strand is 20 nt in length.
6. The RNA complex of sentence 1 or 2, wherein the antisense strand is 21 nt in length.
7. The RNA complex of sentence 1 or 2, wherein the antisense strand is at least 24 nt in length.
8. The RNA complex of sentence 6, wherein the antisense strand is 24 to 121 nt in length.
9. The RNA complex of sentence 1 or 2, wherein the antisense strand is 31 nt in length.
10. The RNA complex of any one of sentences 1 to 9, wherein the sense strand is 15 nt in length.
11. The RNA complex of any one of sentences 1 to 9, wherein the sense strand is 16 nt in length.
12. The RNA complex of any one of sentences 1 to 9, wherein the sense strand is 17 nt in length.
13. The RNA complex of any one of sentences 1 to 12, wherein the antisense strand has a sequence selected from the antisense strand sequences listed in Table 1, Table 2, Table 3, Table 4, Table 5, Table 6, Table 7, Table 8 and Table 10.
14. The RNA complex of any one of sentences 1 to 13, wherein the sense strand has a sequence selected from the sense strand sequences listed in Table 1, Table 2, Table 3, Table 4, Table 5, Table 6, Table 7, Table 8 and Table 10.
15. The RNA complex of any one of sentences 1 and 3 to 14, wherein the RNA complex is capable of inhibiting MyD88 expression by a cell.
16. The RNA complex of any one of sentences 2 to 14, wherein the RNA complex is capable of inhibiting TLR3 expression by a cell.
17. The RNA complex of any one of sentences 1 to 16, wherein the RNA complex comprises a chemical modification.
18. The RNA complex of sentence 17, wherein the RNA modification is a 2'-O-methylated nucleoside, a phosphorothioate bond or a cholesterol moiety.
19. The RNA complex of sentence 18, wherein the RNA complex comprises a cholesterol moiety.
20. The RNA complex of sentence 19, wherein the cholesterol moiety is attached to the 3' terminus of the sense strand.
21. The RNA complex of any one of sentences 17 to 20, wherein the RNA complex comprises a 2'-O-methylated nucleoside.
22. The RNA complex of sentence 21, wherein the 2'-O-methylated nucleoside is positioned at the 3' terminus of the sense strand.
23. The RNA complex of sentence 21, wherein the 3' terminal region of the sense strand comprises a plurality of 2'-O-methylated nucleosides.
24. The RNA complex of sentence 21, wherein the 2'-O-methylated nucleoside is positioned at the 3' terminus of the antisense strand.
25. The RNA complex of sentence 21, wherein the 3' terminal region of the antisense strand comprises a plurality of 2'-O-methylated nucleosides.
26. The RNA complex of sentence 21, wherein a 2'-O-methylated nucleoside is positioned at the 3' terminus of the sense strand and at the 3' terminus of the antisense strand.
27. The RNA complex of sentence 21, wherein the 3' terminal region of the sense strand comprises a plurality of 2'-O-methylated nucleosides and the 3' terminal region of the antisense strand comprises a plurality of 2'-O-methylated nucleosides.
28. The RNA complex of any one of sentences 17 to 27, wherein the RNA complex comprises a phosphorothioate bond.
29. The RNA complex of sentence 28, wherein at least 25% of the bonds between the ribonucleotides in the sense strand of the RNA complex are phosphorothioate bonds.
30. The RNA complex of sentence 28, wherein at least 50% of the bonds between the ribonucleotides in the sense strand of the RNA complex are phosphorothioate bonds.
31. The RNA complex of sentence 28, wherein at least 75% of the bonds between the ribonucleotides in the sense strand of the RNA complex are phosphorothioate bonds.
32. The RNA complex of sentence 28, wherein all of the bonds between the ribonucleotides in the sense strand of the RNA complex are phosphorothioate bonds.
33. The RNA complex of any one of sentences 28 to 32, wherein at least 25% of the bonds between the ribonucleotides in the antisense strand of the RNA complex are phosphorothioate bonds.
34. The RNA complex of any one of sentences 38 to 32, wherein at least 50% of the bonds between the ribonucleotides in the sense strand of the RNA complex are phosphorothioate bonds.
35. The RNA complex of any one of sentences 28 to 32, wherein at least 75% of the bonds between the ribonucleotides in the sense strand of the RNA complex are phosphorothioate bonds.
36. The RNA complex of any one of sentences 28 to 32, wherein all of the bonds between the ribonucleotides in the sense strand of the RNA complex are phosphorothioate bonds.
37. The RNA complex of any one of sentences 28 to 36, wherein the RNA complex is capable of penetrating the cellular membrane of a cell in the absence of a delivery vehicle.
38. The RNA complex of any one of sentences 1 to 37, wherein the RNA complex is not cytotoxic.
39. A method of inhibiting MyD88 or TLR3 expression by a cell comprising contacting the cell with an RNA complex of any one of claims 1 to 38.
40. The method of sentence 39, wherein the cell is present in the eye of a human subject.
41. The method of sentence 39, wherein the subject has AMD.
42. The method of sentence 41, wherein the AMD is wet AMD.
43. The method of sentence 42, wherein the AMD is dry AMD.
44. The method of any one of sentences 40 to 43, comprising administering the RNA complex to the eye of the subject.
45. The method of sentence 44, wherein the RNA complex is administered by intravitreal injection.
46. A method of treating AMD in a subject comprising administering to the subject an RNA complex of any one of claims 1 to 38.
47. The method of sentence 46, wherein the AMD is wet AMD.
48. The method of sentence 46, wherein the AMD is dry AMD.
49. The method of any one of sentences 46 to 48, comprising administering the RNA complex to the eye of the subject.
50. The method of sentence 49, wherein the RNA complex is administered by intravitreal injection.
51. A pharmaceutical composition comprising an RNA complex of any one of sentences 1 to 38 and a pharmaceutically acceptable carrier.
52. The pharmaceutical composition of sentence 51, wherein the composition is intravitreal delivery.
53. The pharmaceutical composition of sentence 51, wherein the pharmaceutical composition is an eye drop.
54. A method of treating AMD in a subject comprising administering to the subject a pharmaceutical composition of any one of sentence 51 to 53.
55. The method of sentence 54, wherein the AMD is wet AMD.
56. The method of sentence 54, wherein the AMD is dry AMD.
57. The method of any one of sentences 54 to 56, comprising administering the pharmaceutical composition to the eye of the subject.
58. The method of sentence 57, wherein the pharmaceutical composition is administered by intravitreal injection.

## Claims

1. An RNA complex comprising an antisense strand of at least 19 nucleotides (nt) in length having sequence complementarity to a MyD88 mRNA sequence and a sense strand of 15 to 17 nt in length having sequence complementarity to the antisense strand, wherein the antisense strand and the sense strand form a complex in which the 5' end of the antisense strand and the 3' end of the sense strand form a blunt end.

2. An RNA complex comprising an antisense strand of at least 19 nucleotides (nt) in length having sequence complementarity to a TLR3 mRNA sequence and a sense strand of 15 to 17 nt in length having sequence complementarity to the antisense strand, wherein the antisense strand and the sense strand form a complex in which the 5' end of the antisense strand and the 3' end of the sense strand form a blunt end.

3. The RNA complex of claim 1 or 2, wherein the antisense strand is 19 to 21 nt, or at least 24 in length.

4. The RNA complex of any one of claims 1 to 3, wherein the antisense strand has a sequence selected from the antisense strand sequences listed in Table 1, Table 2, Table 3, Table 4, Table 5, Table 6, Table 7, Table 8 and Table 10.

5. The RNA complex of any one of claims 1 to 4, wherein the sense strand has a sequence selected from the sense strand sequences listed in Table 1, Table 2, Table 3, Table 4, Table 5, Table 6, Table 7, Table 8 and Table 10.

6. The RNA complex of any one of claims 1 to 5, wherein the RNA complex is capable of inhibiting MyD88 or TLR3 expression by a cell.

7. The RNA complex of any one of claims 1 to 6, wherein the RNA complex comprises a chemical modification, and wherein the chemical modification is optionally selected from a 2'-0-methylated nucleoside, a phosphorothioate bond, and a cholesterol moiety.

8. The RNA complex of claim 7, wherein the RNA complex comprises a cholesterol moiety, and wherein the cholesterol moiety is optionally attached to the 3' terminus of the sense strand.

9. The RNA complex of claim 7, wherein
a 2'-0-methylated nucleoside is positioned at the 3' terminus of the sense strand; or the 3' terminal region of the sense strand comprises a plurality of 2'-0-methylated nucleosides; or
a 2'-0-methylated nucleoside is positioned at the 3' terminus of the antisense strand; or
the 3' terminal region of the antisense strand comprises a plurality of 2'-0-methylated nucleosides; or
a 2'-0-methylated nucleoside is positioned at the 3' terminus of the sense strand and at the 3' terminus of the antisense strand; or
the 3' terminal region of the sense strand comprises a plurality of 2'-0-methylated nucleosides and the 3' terminal region of the antisense strand comprises a plurality of 2'-0-methylated nucleosides.

10. The RNA complex of any one of claims 7 to 9, wherein the RNA complex comprises a phosphorothioate bond, and wherein
at least 25%, 50%, 75%, or all of the bonds between the ribonucleotides in the sense strand of the RNA complex are phosphorothioate bonds; or
at least 25%, 50%, 75%, or all of the bonds between the ribonucleotides in the antisense strand of the RNA complex are phosphorothioate bonds.

11. The RNA complex of claim 10, wherein the RNA complex is capable of penetrating the cellular membrane of a cell in the absence of a delivery vehicle, and wherein optionally the RNA complex is not cytotoxic.

12. An RNA complex of any one of claims 1 to 11 for use in inhibiting MyD88 or TLR3 expression by a cell comprising contacting the cell with the RNA complex.

13. The RNA complex for the use of claim 12, wherein the RNA complex is for administration to the eye of the subject, and wherein the RNA complex is optionally for administration by intravitreal injection.

14. An RNA complex of any one of claims 1 to 11 for use in treating AMD, wherein the AMD is optionally wet AMD or dry AMD.

15. A pharmaceutical composition comprising an RNA complex of any one of claims 1 to 11 and a pharmaceutically acceptable carrier, wherein
the composition is optionally intravitreal delivery; and/or
the pharmaceutical composition is optionally an eye drop.
